(19) Europäisches Patentamt
European Patent Office
Office européen des brevets

(11) **EP 2 094 710 B1**

(12) **EUROPÄISCHE PATENTSCHRIFT**

(45) Veröffentlichungstag und Bekanntmachung des Hinweises auf die Patenterteilung:
**26.05.2010 Patentblatt 2010/21**

(21) Anmeldenummer: **07846949.1**

(22) Anmeldetag: **01.12.2007**

(51) Int Cl.:
***C07D 487/06*** *(2006.01)*

(86) Internationale Anmeldenummer:
**PCT/EP2007/010460**

(87) Internationale Veröffentlichungsnummer:
**WO 2008/067965 (12.06.2008 Gazette 2008/24)**

(54) **3,4 DIAMINOPYRIDIN-DERIVATE**

3,4 DIAMINOPYRIDINE DERIVATIVES

DÉRIVÉS DE 3,4 DIAMINOPYRIDINE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **06.12.2006 DE 102006057580**

(43) Veröffentlichungstag der Anmeldung:
**02.09.2009 Patentblatt 2009/36**

(73) Patentinhaber:
- **Bayer MaterialScience AG**
  **51368 Leverkusen (DE)**
- **Ludwig-Maximilians-Universität München**
  **80539 München (DE)**

(72) Erfinder:
- **ZIPSE, Hendrik**
  **82131 Gauting (DE)**
- **HELD, Ingmar**
  **81377 München (DE)**

(74) Vertreter: **Klimiuk, Meike et al**
**Bayer MaterialScience AG**
**LP/PL, Q 18**
**51368 Leverkusen (DE)**

(56) Entgegenhaltungen:
- **HELD, INGMAR ET AL: "Modular design of pyridine-based acyl-transfer catalysts" SYNTHESIS , (8), 1185-1196 CODEN: SYNTBF; ISSN: 0039-7881, 2007, XP002473757**
- **ANGEWANDTE CHEMIE INTERNATIONAL EDITION IN ENGLISH VOLUME 8, ISSUE 12 , PAGES 981 - 981; DOZ. DR. W. STEGLICH, DR. G. HÖFLE: N,N-DIMETHYL-4-PYRIDINAMINE, A VERY EFFECTIVE ACYLATION CATALYST, 17. Dezember 2003 (2003-12-17), XP002473758 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft 3,4-Diaminopyridin-Derivate, ein Verfahren zu deren Herstellung sowie die Verwendung der 3,4-Diaminopyridin-Derivate.

Donor-substituierte Pyridine spielen in einer Vielzahl synthetisch wichtiger Transformationen wie der Acylierung von Alkoholen, Aminen oder Enolaten eine herausragende Rolle als nukleophile Katalysatoren.[1,2]

In letzter Zeit konnten in kinetischen Racematspaltungs-Experimenten mit entsprechend substituierten Derivaten von (4-Dimethylamino)pyridin **1** (DMAP) oder (4-Pyrrolidino)pyridin **2** (PPY) (siehe Schema 1) große Fortschritte erzielt werden.[2-8] Trotz dieser Entwicklungen bleibt das Feld insgesamt etwas unausgeglichen, bieten sich doch für ausgesuchte synthetische Probleme, wie etwa die kinetische Racematspaltung sekundärer Alkohole, vielfältige Lösungswege an, während andere, scheinbar ähnlich gelagerte Herausforderungen, wie etwa die kinetische Racematspaltung primärer oder tertiärer Alkohole, kaum lösbar erscheinen.

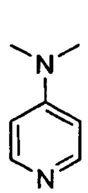
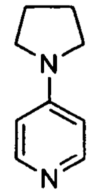

**1** **2**

**Schema 1: Strukturformeln von (4-Dimethylamino)pyridin 1 und (4-Pyrrolidino)pyridin 2.**

In dieser Situation scheint ein modulares Katalysator-Konzept höchst erstrebenswert, das im Rahmen einer einzigen Synthesestrategie eine breite strukturelle Variationsvielfalt ermöglicht. Konzepte dieser Art wurden in jüngster Zeit von Miller et al. mit Katalysatoren erprobt, die auf peptidischen Strukturen basieren und Imidazol als aktives Zentrum verwenden.[2d,9] Peptide sind auch das variable Strukturelement in PPY-Derivaten, die von Kawabata et al. und von Campbell et al. entwickelt wurden.[3,4] In all diesen Fällen beeinflussen die peptidischen Strukturen den Reaktionsverlauf durch zusätzliche Kontakte, die zwischen Substraten und den Seitenketten im geschwindigkeitsbestimmenden Schritt des Katalysezyklus aufgebaut werden. Der elektronische Charakter des nukleophilen Katalyse-Zentrums wird durch die Variationen der Peptidstrukturen in der Regel jedoch nicht verändert.

Der modulare Aufbau von 3-substituierten Derivaten von **1** und **2** hat hingegen das Potential sowohl die Nucleophilie des Pyridinrings als auch dessen Seiten-Asymmetrie zu modifizieren.[4-8] Bisher durchgeführte Experimente beschränken sich auf die Untersuchung stereodifferenzierender Prozesse.

Frühere Studien zum katalytischen Potential Donor-substituierter Pyridine in Acylierungsreaktionen haben bereits auf die relative Stabilität von Acylpyridinium-Kationen **4Ac** als qualitativem Kriterium für die katalytische Aktivität hingewiesen.[10,11]

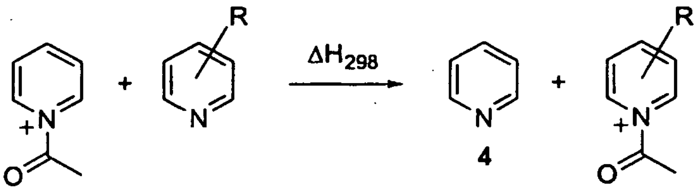


**4** (1)

**4Ac**

**Schema 2: Acyl-Transferreaktion (1) von einem Acylpyridinium-Kation auf ein substituiertes Pyridin.**

Der vorliegenden Erfindung liegt daher die Aufgabe zugrunde, Verbindungen bereitzustellen, die als Katalysator im Rahmen einer einzigen Synthesestrategie eine breite strukturelle Variationsvielfalt ermöglichen. Es ist eine weitere Aufgabe der vorliegenden Erfindung, Verbindungen bereitzustellen, die in einer breiten strukturellen Variationsvielfalt

einfach herzustellen sind. Insbesondere wäre es wünschenswert, Verbindungen zu kennen, die als Katalysatoren für Acylierungsreaktionen und zur Herstellung von Urethanen und Polyurethanen verwendet werden können.

Die oben genannten Aufgaben werden durch den Gegenstand der unabhängigen Ansprüche gelöst. Bevorzugte Ausführungsformen ergeben sich aus den Unteransprüchen.

Überaschenderweise haben die Erfinder der vorliegenden Erfindung festgestellt, dass 3,4-Diaminopyridin-Derivate leicht herzustellen sind und vorteilhafte Eigenschaften als Katalysator besitzen.

Ein erster Aspekt der vorliegenden Erfindung betrifft ein 3,4-Diaminopyridin-Derivat der folgenden Formel **I**

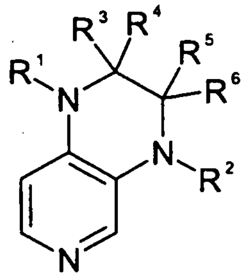

wobei

$R^1$ und $R^2$ jeweils unabhängig voneinander Elektronendonoren sind, wobei $R^2$ auch H sein kann; und

$R^3$, $R^4$, $R^5$ und $R^6$ jeweils unabhängig voneinander ausgewählt sind aus H, substituiertem oder unsubstituiertem, geradkettigen oder verzweigten Alkyl, Alkenyl, Alkinyl, Alkoxy und substituiertem oder unsubstituiertem Aryl, wobei die Reste $R^3$ und/oder $R^4$ mit den Resten $R^5$ und/oder $R^6$ einen Ring bilden können.

Insbesondere sind die 3,4-Diaminopyridin-Derivate der vorliegenden Erfindung durch die breite Variation der elektronischen Eigenschaften des Pyridin-Rings als vielseitig einsetzbarer Katalysator geeignet. Durch die verbrückende Ethylen-Einheit zwischen den beiden Stickstoffatomen in 3- und 4-Position des Pyridinrings kann eine vorteilhafte Elektronenschiebende Wirkung auf den Pyridinring ausgeübt werden. Die Ethylenbrücke kann ein oder mehrfach substituiert sein. Dabei ist zumindest das Stickstoffatom an der 4-Position am Pyridinring vollständig substituiert, d.h. es trägt kein Wasserstoffatom mehr.

Gemäß einer bevorzugten Weiterbildung der vorliegenden Erfindung wird ein 3,4-Diaminopyridin-Derivat gemäß Formel I offenbart, wobei die Elektronendonoren $R^1$ und $R^2$ jeweils unabhängig voneinander ausgewählt sind aus substituiertem oder unsubstituiertem, geradkettigem oder verzweigten Alkyl, Alkenyl, Alkinyl, Acyl, Alkoxy und substituiertem oder unsubstituiertem Aryl, bevorzugter substituiertem oder unsubstituiertem Phenyl und Benzyl, wobei $R^2$ auch H sein kann.

Unter einem Elektronendonor soll hier ein Substituent verstanden werden, der die Elektronendichte an den jeweiligen Substitutionszentren erhöhen. Die Reste $R^1$ und $R^2$ erhöhen hier die Elektronendichte an den Stickstoffatomen an den Positionen 3 und 4 des Pyridinrings.

Unter den oben genannten Substituenten Alkyl, Alkenyl, Alkinyl, Acyl und Alkoxy soll jeweils unabhängig voneinander das Folgende verstanden werden.

Unter Alkyl soll ein gesättigter Kohlenwasserstoffrest verstanden werden. Bei dem Alkyl handelt es sich bevorzugt um substituiertes oder unsubstituiertes, geradkettiges oder verzweigtes $C_1$-$C_{20}$-Alkyl, bevorzugter um $C_1$-$C_{10}$-Alkyl, weiter bevorzugt um $C_1$-$C_8$-Alkyl, noch weiter bevorzugt um $C_2$-$C_6$-Alkyl und am bevorzugtesten um $C_3$-$C_5$-Alkyl. Beispiele solcher Alkylsubstituenten sind Methyl, Ethyl, n-Propyl, iso-Propyl, n-Butyl, sec-Butyl und t-Butyl.

Unter Alkenyl soll ein einfach oder mehrfach ungesättigter Rest verstanden werden, d.h. ein Alkylrest mit einer oder mehreren Doppelbindungen. Bei dem Alkenyl handelt es sich bevorzugt um substituiertes oder unsubstituiertes, geradkettiges oder verzweigtes $C_2$-$C_{20}$-Alkenyl, bevorzugter um $C_2$-$C_{10}$-Alkenyl, weiter bevorzugt um $C_2$-$C_8$-Alkenyl, noch weiter bevorzugt um $C_3$-$C_6$-Alkenyl und am bevorzugtesten um $C_3$-$C_5$-Alkenyl.

Unter Alkinyl soll ein einfach oder mehrfach mit Dreifachbindungen ungesättigter Rest verstanden werden, d.h. ein Alkylrest mit einer oder mehreren Dreifachbindungen. Dabei kann der Rest zusätzlich eine oder mehrere Doppelbindungen aufweisen. Bei dem Alkinyl handelt es sich bevorzugt um substituiertes oder unsubstituiertes, geradkettiges oder verzweigtes $C_2$-$C_{20}$-Alkinyl, bevorzugter um $C_2$-$C_{10}$Alkinyl, weiter bevorzugt um $C_2$-$C_8$-Alkinyl, noch weiter bevorzugt um $C_3$-$C_6$-Alkinyl und am bevorzugtesten um $C_3$-$C_5$-Alkinyl.

Unter Acyl soll eine Gruppe -C(O)-R verstanden werden, wobei R ein Alkyl, Alkenyl, Alkinyl oder Aryl wie oben beschrieben ist. Bevorzugt ist Acyl ein substituiertes oder unsubstituiertes, geradkettiges oder verzweigtes $C_1$-$C_{20}$-Acyl, bevorzugter ein $C_1$-$C_{10}$-Acyl, weiter bevorzugt ein $C_1$-$C_8$-Acyl, noch weiter bevorzugt ein $C_2$-$C_6$-Acyl und am bevorzugtesten ein $C_3$-$C_5$-Acyl, oder Benzoyl. Ein insbesondere bevorzugter Acyl-Rest ist der Acetyl-Rest.

Unter Alkoxy soll ein über ein Sauerstoffatom gebundener Alkylrest verstanden werden. Bei dem Alkoxy handelt es sich bevorzugt um substituiertes oder unsubstituiertes, geradkettiges oder verzweigtes $C_1$-$C_{20}$-Alkoxy, bevorzugter um $C_1$-$C_{10}$-Alkoxy, weiter bevorzugt um $C_1$-$C_8$-Alkoxy, noch weiter bevorzugt um $C_2$-$C_6$-Alkoxy und am bevorzugtesten um $C_3$-$C_5$-Alkoxy. Beispiele solcher Alkylsubstituenten sind Methoxy, Ethoxy, n-Propoxy, iso-Propoxy, n-Butoxy, sec-Butoxy und t-Butoxy.

Gemäß einer anderen Weiterbildung der vorliegenden Erfindung ist mindestens einer der Reste $R^3$, $R^4$, $R^5$ und $R^6$ nicht H. Bevorzugt ist dabei mindestens einer der Reste $R^3$, $R^4$, $R^5$ und $R^6$ Phenyl. Weiter bevorzugt sind die beiden Reste $R^3$ und $R^6$ H und die beiden Reste $R^4$ und $R^5$ gemeinsam 1,4-Butandiyl, um einen 6-Ring auszubilden, wie er beispielsweise in der Verbindung **11** unten gezeigt ist. Es ist jedoch auch möglich, dass dieser 6-Ring ein ungesättigter oder substituierter Ring ist.

Gemäß noch einer anderen bevorzugten Weiterbildung der vorliegenden Erfindung wird ein 3,4-Diaminopyridin-Derivat gemäß Formel I offenbart, wobei die beiden Reste $R^4$ und $R^5$ in einer cis-Stellung zueinander angeordnet sind, um ein 3,4-Diaminopyridin-Derivat gemäß Formel Ia zu ergeben:

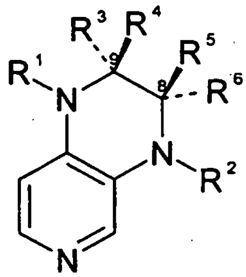

**Ia**

wobei
$R^1$ und $R^2$ jeweils unabhängig voneinander Elektronendonoren sind, wobei $R^2$ auch H sein kann;
$R^3$ und $R^6$ jeweils unabhängig voneinander ausgewählt sind aus H, substituiertem oder unsubstituiertem, geradkettigen oder verzweigten Alkyl, Alkenyl, Alkinyl, Alkoxy und substituiertem oder unsubstituiertem Ary;
$R^4$ und $R^5$ jeweils unabhängig voneinander ausgewählt sind aus substituiertem oder unsubstituiertem, geradkettigen oder verzweigten Alkyl, Alkenyl, Alkinyl, Alkoxy und substituiertem oder unsubstituiertem Aryl; und
wobei die Reste $R^3$ und/oder $R^4$ mit den Resten $R^5$ und/oder $R^6$ einen Ring bilden können.

Gemäß dieser Weiterbildung sind die beiden Reste $R^4$ und $R^5$ in einer cis-Stellung bezüglich einer Ebene angeordnet, die durch die Atome des Pyridinrings, die beiden Stickstoffatome an den Positionen 3 und 4 des Pyridinrings sowie die verbrückende Ethyleneinheit mit den Kohlenstoffatomen 8 und 9 gebildet wird. Liegen mehrere Substituenten an den Kohlenstoffatomen 8 und 9 vor, d.h. mindestens zwei der Reste $R^3$ und $R^4$ oder $R^5$ und $R^6$ sind nicht H, ist es besonders bevorzugt, wenn die Reste $R^4$ und $R^5$ einen Ring bilden, dass dieser in cis-Stellung vorliegt. Liegt keine Ringbildung vor, ist es bevorzugt, wenn die beiden sterisch anspruchsvolleren Substituenten der Reste $R^3$, $R^4$, $R^5$ und $R^6$ eine cis-Stellung an den Kohlenstoffatomen 8 und 9 einnehmen.

Bei einem insbesondere bevorzugten 3,4-Diaminopyridin-Derivat ist $R^1$ und $R^2$ jeweils Ethyl, $R^3$ und $R^5$ jeweils H und $R^4$ und $R^6$ gemeinsam 1,4-Butandiyl, um einen 6-Ring auszubilden. Diese Verbindung ist in der vorliegenden Anmeldung mit **11** bezeichnet.

Gemäß noch einer anderen bevorzugten Weiterbildung der vorliegenden Erfindung sind $R^1$ und $R^2$ jeweils unabhängig voneinander ein substituiertes oder unsubstituiertes, geradkettiges oder verzweigtes $C_1$-$C_{10}$-Alkyl, bevorzugt ein substituiertes oder unsubstituiertes, geradkettiges oder verzweigtes $C_2$-$C_8$-Alkyl und am bevorzugtesten ein substituiertes oder unsubstituiertes, geradkettiges oder verzweigtes $C_3$-$C_6$-Alkyl.

Ein zweiter Aspekt der vorliegenden Erfindung betrifft ein Verfahren zur Herstellung eines 3,4-Diaminopyridin-Derivates gemäß Formel I mit den Schritten:

- Umsetzen von 3,4-Diaminopyridin mit einer 1,2-Dicarbonylverbindung;
- Reduzieren des resultierenden Diimins zu dem korrespondierenden Diamin; und
- Zumindest Substituieren des Wasserstoffatoms am Stickstoff an der 4-Position des Pyridinrings.

Bei der 1,2-Dicarbonylverbindung handelt es sich bevorzugt um eine Verbindung der unten dargestellten Formel

II

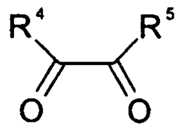

II

Gemäß einer bevorzugten Weiterbildung des zweiten Aspekts werden beide Stickstoffatome an den Positionen 3 und 4 des Pyridinrings substituiert.

Bevorzugt kann eine Alkyl-Substitution an den Stickstoffatomen der Positionen 3 und/oder 4 des Pyridinrings über eine zweistufige Synthese mit einer Acylierung des Stickstoffatoms und einer anschließenden Reduktion des resultierenden Amids zu einem Amin bewirkt werden. Sollen weitere Substituente $R^3$ und $R^6$ eingeführt werden, erfolgt dies bevorzugt während der Reduktion des Diimins mittels anionischer Alkylgruppen.

Ein dritter Aspekt der vorliegenden Erfindung betrifft die Verwendung eines 3,4-Diaminopyridins gemäß Formel I oben als Katalysator bei einer chemischen Umsetzung. Bevorzugt ist diese chemische Umsetzung eine Acylierungsreaktion, insbesondere eine Acylierungsreaktion eines Alkohols oder eines Amins, oder eine Urethan- und/oder Polyurethansynthese ist. Die 3,4-Diaminopyridine der vorliegenden Erfindung können vorteilhafterweise die Reaktion eines Isocyanates mit einem Alkohol katalysieren. Daher sind die 3,4-Diaminopyridine der vorliegenden Erfindung bei der Synthese von Urethanen und/oder Polyurethanen nützlich.

Gemäß der üblichen Verwendung eines Katalysators werden die 3,4-Diaminopyridine der vorliegenden Erfindung zu den für die zu katalysierende Reaktion notwendigen Reagenzien und Lösungsmittel(n) zugegeben. Dies können die für eine Acylierungsreaktion oder eine Urethan- und/oder Polyurethanherstellung notwendigen Reagenzien sein.

Die Verbindungen der vorliegenden Erfindung können auch vorteilhaft zur Herstellung eines pharmazeutischen Mittels oder eines Herbizids verwendet werden.

Im Folgenden soll die vorliegende Erfindung anhand von Beispielen mit Verweis auf die begleitenden Figuren näher beschrieben werden, in denen

**Fig. 1** die Reaktionsenthalpien für 3,4-Diaminopyridine 6-21 im Vergleich zu den Pyridinen **1, 2** und **5** gemäß Gleichung (1) aus Schema 1 oben; und

**Fig. 2** die Einkristall-Röntgenstruktur der Verbindung **34** zeigt.

Die Entwicklung von Katalysatoren, die auf dem 3,4-Diaminopyridin-Motiv basieren, wurden durch eine theoretische Abschätzung der Stabilität der entsprechenden Acyl-Intermediate gestützt, die quantitativ durch die Reaktionsenthalpie der homodesmischen Acyl-Transferreaktion (1) (siehe Schema 2) ausgedrückt werden kann.

Die Reaktionsenthalpien für eine Vielzahl von 3,4-Diaminopyridinen sind in Tabelle 1 zusammen mit den Werten für andere Pyridin-Derivate zusammengestellt, deren katalytisches Potential bereits aus früheren Studien bekannt ist. Hierzu zählen Pyridin **(4),** DMAP **(1),** PPY **(2)** und das trizyklische DMAP-Derivat **5,** die alle nicht gemäß der vorliegenden Erfindung sind. Eine graphische Darstellung der in Tabelle 1 dargestellten Ergebnisse findet sich in **Fig. 1.**

**Tabelle 1**

| System | $\Delta H_{rxn}(298)$ [kJ $mol^{-1}$] | Ladung q(Ac)[a,b] | Abstand r(C-N)[b] [pm] |
|---|---|---|---|
| **4** | 0.0 | +0.366 | 153.38 |
| **18** | -39.0 | +0.318 | 149.70 |
| **14** | -46.2 | +0.321 | 149.96 |
| **8** | -48.7 | +0.324 | 150.09 |
| **17** | -57.3 | +0.294 | 148.71 |
| **7** | -67.1 | +0.310 | 149.26 |

(fortgesetzt)

| System | $\Delta H_{rxn}(298)$ [kJ mol$^{-1}$] | Ladung q(Ac)[a,b] | Abstand r(C-N)[b] [pm] |
|---|---|---|---|
| **13** | -73.1 | +0.305 | 148.99 |
| **16** | -82.0 | +0.299 | 148.71 |
| **1** | -82.1 | +0.298 | 148.16 |
| **21** | -85.5 | +0.301 | 148.60 |
| **2** | -93.1 | +0.291 | 147.86 |
| **12** | -103.2 | +0.291 | 148.18 |
| **6** | -105.0 | +0.284 | 147.66 |
| **5** | -108.9 | +0.279 | 147.07 |
| **20** | -115.5 | +0.278 | 147.31 |
| **19** | -116.5 | +0.274 | 147.16 |
| **10** | -117.9 | +0.277 | 147.46 |
| **9** | -119.2 | +0.277 | 147.29 |
| **15** | -122.2 | +0.274 | 147.12 |
| **11** | -127.1 | +0.272 | 147.06 |

**Tabelle 1.** Reaktionsenthalpien $\Delta H_{rxn}(298)$ für 298.15 K für die Acetyl-Übertragungsreaktion gemäß Gleichung (1) aus Schema 1, die auf B3LYP/6-311+G(d,p)// B3LYP/6-31G(d)-Niveau berechnet wurden (in kJ mol$^{-1}$).[a] in Einheiten der Elementarladung *e*; [b] Ladungs- und Distanz-Parameter beziehen sich immer auf das günstigste Konformer.

Das strukturell einfachste hier berücksichtigte 3,4-Diaminopyridin ist **6,** das auf dem Tetrahydropyrido[3,4-b] pyrazin-Gerüst basiert. Die Stabilität des entsprechenden Acetyl-Intermediats **6Ac** liegt nahe bei der des katalytisch hochaktiven **5.** Austausch der beiden Methylgruppen in **6** gegen eine (in **7** und **21)** oder zwei (wie in **8)** Acetylgruppen verringert die Elektronendichte im Pyridin-Ring und somit auch die Stabilität der entsprechenden Kationen **7Ac** und **8Ac** recht deutlich. Die Stabilitäts-Differenz zwischen **6Ac** und **21Ac** (19.5 kJ mol$^{-1}$) und zwischen **6Ac** und **7Ac** (37.9 kJ mol$^{-1}$) zeigt, dass der Stickstoff in 4-Position deutlich sensibler auf Änderungen des Substitutionsmusters reagiert als der Stickstoff an 3-Position. Der kombinierte Effekt beider Substituenten in **8** (56.3 kJ mol$^{-1}$ relativ zu **6**) ist praktisch identisch zur Summe der beiden Einzel-Substitutionen (57.4 kJ mol$^{-1}$). Austausch der beiden Methylgruppen in **6** gegen Ethyl-Gruppen erhöht die Stabilität des Acetyl-Intermediats um 10 kJ mol$^{-1}$. Anellierung eines gesättigten Alkylrings an System **6** in entweder *cis*-Orientierung (wie in 9) oder trans-Orientierung (wie in **10)** erhöht die Stabilität der Acetyl-Intermediate ebenfalls um ca. 15 kJ mol$^{-1}$. Die Stabilität der Acetylpridinium-Kationen, die sich von den trizyklischen Systemen **9** und **10** ableiten, hängt von den N-Substituenten in ähnlicher Weise ab, wie bereits für das bizyklische System **6.** Einführung von zwei Ethyl-Gruppen führt somit zu den stabilsten Acetyl-Intermediaten **11Ac** und **15Ac.** Schließlich erhöhen auch die Einführung von Aryl-Substituenten in der Ethylenbrücke von **6** (wie etwa in **19)** die Stabilität des entsprechenden kationischen Intermediats, was wahrscheinlich auf induktive Elektronenübertragung auf die 3,4-Diamino-Stickstoffatome zurückzuführen ist. Die Stabilitäts-Werte der Acetyl-Intermediate der Verbindungen in **Fig. 1** decken einen Bereich von fast 90 kJ mol$^{-1}$ ab. Für die Verbindungen **1, 2** und **5** wurde gefunden, dass diese Stabilitätswerte zur katalytischen Aktivität in Beziehung stehen. Ohne dabei an eine Theorie gebunden sein zu wollen, wäre zu erwarten, dass die Stabilitätswerte der 3,4-Diaminopyridine **18** und **11** (die die jeweiligen Enden der Skala markieren) sich in ihrer katalytischen Aktivität um einen Faktor 30 unterscheiden. Ein Blick auf die Ladungs- und Abstands-Daten in Tabelle 1 zeigt weiterhin, dass größere thermodynamische Stabilität mit kürzeren C-N-Bindungsabständen und kleineren Acetylgruppen-Ladungen korreliert.

Einige der in **Fig. 1** gezeigten Verbindungen können in einer 3- bis 5-stufigen Sequenz effizient aus 3,4-Diaminopyridin **22** hergestellt werden (siehe Schema 3). Kondensation von **22** mit Glyoxal, 1,2-Cyclohexadion oder Benzil führen in guten Ausbeuten zu den Diiminen **23 - 25**.[12] Pyridopyrazin **23** (R=H) konnte anschliessend zum Tetrahydro-Derivat **26** reduziert[13] und unter Eschweiler-Clark-Bedingungen zur Verbindung **6** alkyliert werden.[14] Die Einführung längerer Alkylketten gelingt effizienter durch eine zweistufige Sequenz aus einleitender Bis-Acylierung und anschliessender Reduktion zum entsprechenden Diamin.[15] Ausgehend von **26** konnte in dieser Weise die diethylierte Verbindung **20** in 48 % hergestellt werden. Ein ähnliches Vorgehen erlaubt die Synthese des Cyclohexyl-anellierten

Systems **11,** das vom Diamin **27** in zwei Schritten in 37 % Ausbeute erhalten wird. Das *cis*-anellierte Diamin **27** ist aus **24** durch selektive Reduktion mit $NaBH_4/BH_3/H_2NCH_2CH_2OH$ in 74 % Ausbeute[16] oder durch Reduktion mit $LiAlH_3$ bei -40 °C in 90% Ausbeute zugänglich. Die Anwendung des einfacheren Reduktions-Protokolls, das für die Verbindung **26** verwendet wurde, führt in diesem Fall zu einer *cis-*/*trans*-Mischung von **27.**

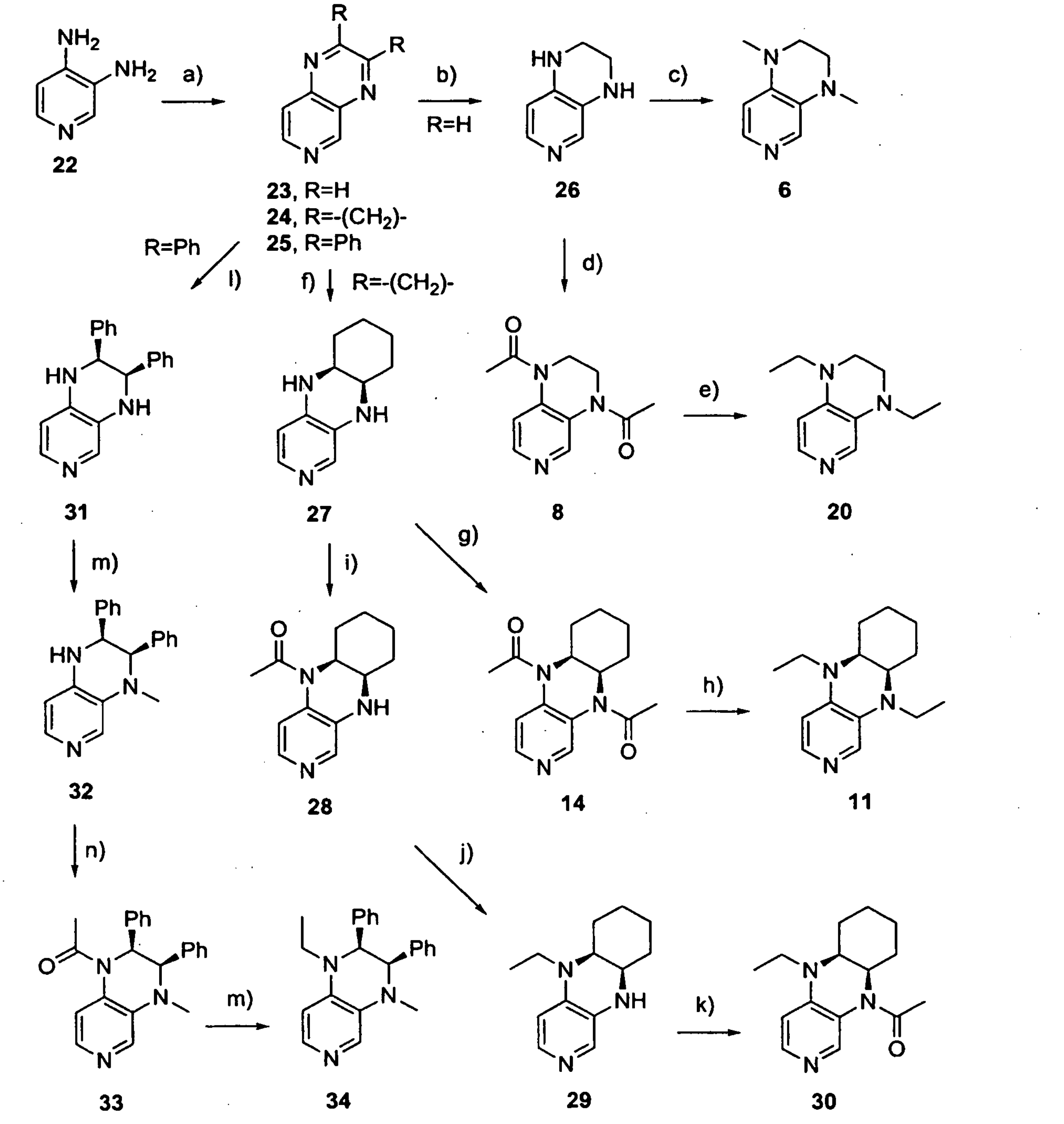


**Schema 3.** Synthese von Katalysatoren basierend auf dem 3,4-Diaminopyridin-Motiv. a) Glyoxal, 1,2-Cylohexadienon, oder Benzil, EtOH, 70 °C, 5h, 95-98 %. b) gepulvertes $NaBH_4$, EtOH, 40 °C, 24 h, 50 %. c) 200 eq. $CH_2O$ (37% in Wasser), 100 eq. $HCO_2H$, 110 °C, 48 h, 57 %. d) $(CH_3CO)_2O$, Pyridin, 100 °C, 24 h, 80 %. e) 4.2 eq. $LiAlH_4$, 2.6 eq. $AlCl_3$, MTBE, 0 °C, 1h, dann Rückfluss, 8 h, 60 %. f) $LiAlH_4$, THF, -40 auf RT, 32 h, 90 %. g) $(CH_3CO)_2O$, 25 mol% PPY, Pyridin, 100 °C, 48 h, 68 %. h) 4.2 eq. $LiAlH_4$, 2.6 eq. $AlCl_3$, MTBE, 0 °C, 1h, dann Rückfluss, 8 h, 55 %. i) 1.1 eq. $(CH_3CO)_2O$, 0.2 eq. 2, $NEt_3$, DCM, RT, 3 h, 82 %. j) 2.2 eq. $LiAlH_4$, 1.3 eq. $AlCl_3$, MTBE, 0 °C, 1h, dann Rückfluss, 8 h, 65 %. k) 1) 1 eq. n-BuLi, THF, -78 °C, 1h, dann 0 °C, 1h; 2) 1.1 eq. $CH_3COCl$, THF, -78 °C bis RT, 1 h, 16%. 1) gepulvertes $NaBH_4$, EtOH, 40 °C, 48 h, 74 %. m) 88 eq. $CH_2O$ (37% in Wasser), 200 eq. $HCO_2H$, 110 °C, 48 h, 90 %. n) 1) 1 eq. n-BuLi, MTBE, -78 °C, 1h, dann 0 °C, 1h; 2) 1.1 eq. $CH_3COCl$, MTBE, -78 bis 0 °C, 1 h, 36%. o) 2.2 eq.

$LiAlH_4$, 1.3 eq. $AlCl_3$, THF, 0 °C, 1h, dann Rückfluss, 18 h, 53 %.

Anwendung eines milderen Acylierungsverfahrens für die Umsetzung von **27** weist auf eine deutlich höhere Reaktivität der C4-Aminogruppe hin, die die bevorzugte Bildung des Amids **28** als einziges Reaktionsprodukt erklärt. Die Reduktion dieses Amids wie zuvor zum entsprechenden Amin und Acylierung der noch verbleibenden Amino-Funktion an C3 erlaubt somit den Aufbau von Katalysatoren, die zwei Donor-Substituenten unterschiedlicher Stärke enthalten. Der Einfluss von Aryl-Substituenten in der Verbindungsbrücke zwischen den beiden Amino-Stickstoffatomen an C3 und C4 wurde durch Synthese der Verbindung **34** untersucht. Während sich die Reduktion des entsprechenden Pyridopyrazins **25** unter den für **26**[13] verwendeten Bedingungen gut durchführen lässt, bleibt die anschließende Eschweiler-Clark-Alkylierung auf der Stufe der Monoalkylierung zur Verbindung **32** stehen. Die Acetylierung von **32** erwies sich unter allen vorher verwendeten Bedingungen als überraschend schwierig, konnte aber letztendlich durch einleitende Deprotonierung mit *n*-BuLi, Abfangen des Amid-Anions mit AcCl und abschließender Reduktion zu **34** durchgeführt werden.

Die Zusammensetzung aller in Schema 3 gezeigten Verbindungen wird durch hochauflösende Massenspektrometrie belegt. Die Struktur des Pyridin-Rings kann in allen Verbindungen direkt aus der Anwesenheit von drei Signalen (einem Singulett, zwei Dubletts) im entsprechenden Bereich des $^{1}$H-NMR-Spektrums (6.5-8 ppm) abgeleitet werden. Somit verbleibt das Problem der stereochemischen Zuordnung der Cyclohexan-Anellierung in den Verbindungen **11, 14** und **27-**30, sowie die regiochemische Kontrolle der N-Alkylierungs- und N-Acylierungs-Reaktionen. Die $^{1}$H-NMR-Signale für die benachbarten Protonen $H_a$ und $H_e$ in Struktur **11** (siehe Schema 4) weisen eine Kopplungskonstante von $^{3}J_{HH}$=2.8 Hz auf und zeigen ein positives NOE-Signal, welches entweder mit einer axial-äqatorial- oder einer äquatorial-äquatorial-Anordnung vereinbar ist.[17] Eine äquatorial-äquatorial-Orientierung der beiden Protonen kann aber ausgeschlossen werden, da die 3,4-Diaminopyridin-Einheit kaum durch diaxiale Anellierung an den Cyclohexanring angebunden sein kann.

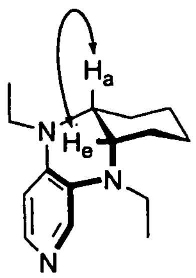

**11**

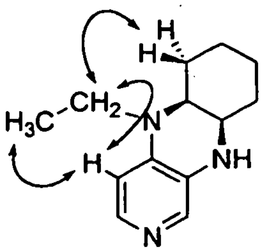

**29**

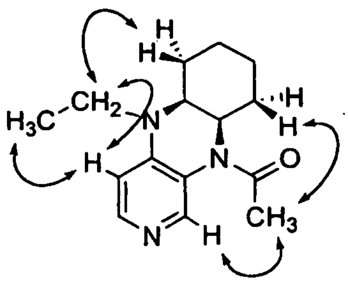

**30**

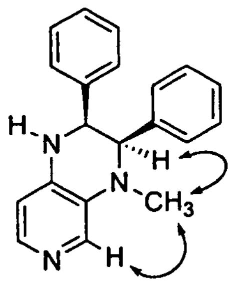

**31**

**Schema 4.** Pfeile weisen auf beobachtete NOE-Verstärkungen hin.

Die Regioselektivität der Acetylierung von **27** zu **28** konnte nicht direkt nachgewiesen werden, da die spektroskopischen Daten von **28** in dieser Hinsicht nicht aussagekräftig waren. Nach Reduktion von **28** zu **29** können jedoch positive NOE-Signale zwischen der Ethylgruppe und dem Proton an C5-Position des Pyridinrings beobachtete werden. Dies stützt die in Schema 3 gezeigte Struktur. Die Regiochemie der N-Methylierung von **31** zu **32** konnte ebenfalls durch die Beobachtung von NOE-Verstärkungen zwischen den Signalen für die Methylgruppe (2.85 ppm) und denen für das Proton an C1 des Pyridinrings (7.82 ppm) wie auch einem der benzylischen Protonen (4.44 ppm) festgelegt werden. Dass diese Regiochemie auch im doppelt alkylierten Produkt **34** noch beibehalten wird konnte zweifelsfrei durch die Einkristall-Röntgenstrukturanalyse dieser Verbindung bestätigt werden (siehe **Fig. 2,** kristallisiert aus Isohexan/Dichlormethan).

## Katalytische Eigenschaften

Die katalytischen Eigenschaften der Pyridine **1, 2, 5, 6, 8, 11, 20** und **34** wurden in zwei Acylierungsreaktionen **A** und **B** untersucht, die in Schema 5 dargestellt sind.

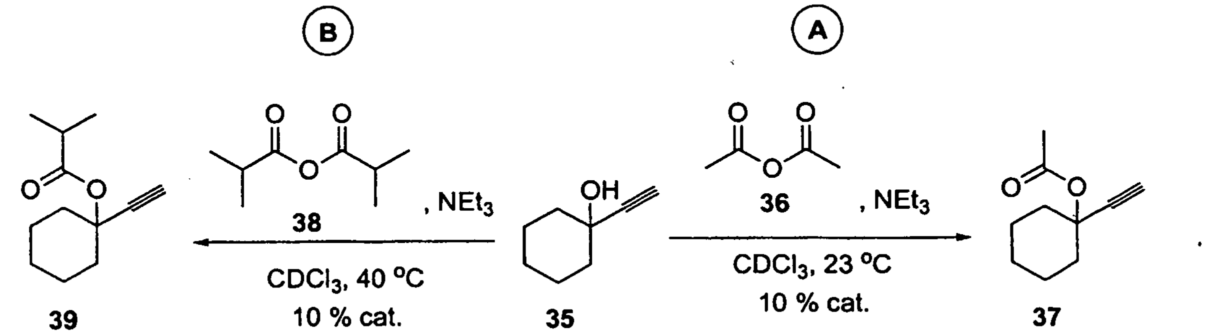


**Schema 5: Darstellung der Acylierungsreaktionen A und B.**

Der tertiäre Alkohol **35** wurde bereits in früheren Studien aufgrund seiner geringen Reaktivität als Modell-Substrat verwendet.[10] Die Acetylierung von **35** mit Essigsäure-Anhydrid (**36**) in der Gegenwart von Triethylamin als Hilfsbase (Reaktion **A**) verläuft quantitativ, solange ein Katalysator in einer Konzentration von 10% (relativ zu **35**) eingesetzt wird, der zumindest die Reaktivität von DMAP (1) aufweist. Die Reaktions-Halbwertszeit $\tau_{1/2}$ kann durch Integration der Signale der Edukte und Produkte im $^1$H-NMR-Spektrum bestimmt werden und wird hier als Maß der katalytischen Aktivität verwendet. In Reaktion **B** wird der gleich Alkohol wie zuvor mit Isobuttersäure-Anhydrid (**38**) als Acylierungsreagenz umgesetzt. Da diese Reaktion deutlich langsamer verläuft als die von **36** wurde für Reaktion eine höhere Reaktionstemperatur (40°C) gewählt. Diese Wahl führt zu näherungsweise ähnlichen Halbwertszeiten für beide Reaktionen.

Basierend auf neueren Untersuchungen von DMAP-katalysierten Acylierungs-Reaktionen[18,19] kann in unpolaren Lösungsmitteln für beide Reaktionen der in Schema 6 gezeigte Mechanismus angenommen werden. Demnach reagieren Anhydrid und Katalysator in einem ersten (meist raschen) vorgelagerten Schritt unter Bildung eines Komplexes aus Acylpyridinium-Kation und Carboxylat-Anion. Dieser Komplex kann in der Regel unter den hier verwendeten Bedingungen nicht im $^1$H-NMR-Spektrum detektiert werden. Wir können deshalb annehmen, dass die entsprechende Gleichgewichtskonstante K für beide Reaktionen **A** und **B** recht klein ist.

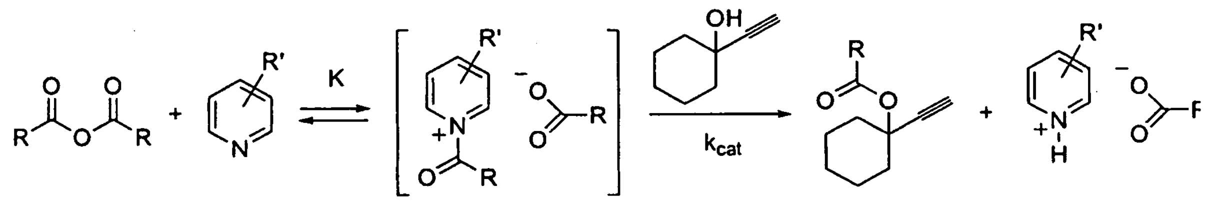


**Schema 6: Mechanismus zur Acylierung gemäß Reaktionen A und B.**

Im geschwindigkeitsbestimmenden Schritt reagiert dann das Acylpyridinium-Kation mit dem Alkohol **35** unter Bildung des Ester-Produkts und des deaktivierten Katalysators. Dieser kann durch Einwirkung der Hilfsbase $NEt_3$ wieder regeneriert werden. Die hier gemessenen Halbwertszeiten $\tau_{1/2}$ (Tabelle 2) reflektieren den Einfluss des Katalysators auf beide diese Schritte. Eine eindeutige Korrelation von $\tau_{1/2}$ mit den berechneten Stabilitätswerten (Tabelle 1) für die Acetylpyridinium-Kationen kann deshalb nur erwartet werden, wenn die Substituenteneffekte auf K größer sind als die auf $k_{cat}$.

**Tabelle 2**

| Katalysator | A $\tau_{1/2}$ (min) | B $\tau_{1/2}$ (min) |
|---|---|---|
| **1 (DMAP)** | 166 | 304 |
| **2 (PPY)** | 75 | 171 |

(fortgesetzt)

| Katalysator | A<br>$\tau_{1/2}$ (min) | B<br>$\tau_{1/2}$ (min) |
|---|---|---|
| **5** | 21 (29) | 67 (86) |
| **6** | 56 | 129 |
| **8** | >38000[a] | >100000[a] |
| **11** | 24 (37) | 63 (81) |
| **20** | 53 | 91 |
| **30** | 138[b] | 112 |
| **34** | 144 | 269 |

**Tabelle 2.** Reaktions-Halbwertszeiten $\tau_{1/2}$ (in min) für die Modell-Reaktionen **A** und **B.** Die Ergebnisse in Klammern wurden unter Verwendung von Hünig-Base (EtN(*i*-Pr)$_2$) als Hilfsbase erhalten.[a] siehe Lit. 20. [b] siehe Lit. 23

Die Reaktivitäts-Daten für die Verbindungen 1, 2 und 5 in Tabelle 2 weichen etwas von denen für die gleiche Reaktion unter identischen Bedingungen in einer früheren Publikation ab.[10] Dies ist teilweise durch geringe Unterschiede in den NMR-Messungen zur Reaktionsverfolgung bedingt (siehe experimenteller Teil). Weiterhin lassen sich die Ergebnisse für aktivere Katalysatoren wie **5** nur bei Verwendung ausserordentlich reiner Proben reproduzieren. Die Durchführung der Reaktion unter strikt anaeroben Bedingungen ist hingegen nicht erforderlich. Die Ergebnisse in Tabelle 2 für die Katalysatoren **6, 11** und **20** zeigen, dass Diaminopyridine deutlich reaktiver sind als einfach substituierte Pyridine wie DMAP oder PPY. Die besten Ergebnisse konnten für Verbindung **11** erhalten werden, die ähnlich aktiv ist wie die bereits bekannte carbozyklische Verbindung **5.** Vergleich der Ergebnisse für **20** und **11** zeigt einen starken Einfluss der Donor-Eigenschaften des Brücken-Motivs, während der Unterschied zwischen N-Methyl- oder N-Ethyl-Substitution **(20** vs. **6)** eher gering ausfällt. Während diese Ergebnisse durchaus in Einklang mit den Stabilitäswerten in Tabelle 1 und Fig. 1 sind, ist die katalytische Aktivität von **34,** gemessen am Stabilitätswert der verwandten Verbindung **19,** überraschend gering. Die katalytische Aktivität wird durch die Einführung von Akzeptor-Substitutenten deutlich reduziert, was durch die geringe Aktivität von **30** (im Vergleich zu **11**), wie auch der extrem langsamen Reaktion bei Verwendung der N-acylierten Verbindung **8** illustriert wird. Während dies mit Blick auf den geringen Stabilitätswert des entsprechenden Acetyl-Intermediats zu erwarten war, müssen wir doch anerkennen, dass die Stabilitäts-Skala in Fig. 1 nicht in allen Fällen in Einklang mit den experimentell gefundenen Reaktivitäts-Daten in Tabelle **2** steht. Dass letztere auch von der Wahl der Substrate abhängen, wird durch Vergleich der Ergebnisse für die Reaktionen **A** und **B** deutlich. Das Reaktivitäts-Verhältnis für die aktivste Verbindung **11** und DMAP beträgt für die Reaktion A 7.0 und für die Reaktion **B** 4.8. Ein ähnliches Ergebnis wird für das Pyridinderivat **5** mit Reaktivitäts-Verhältnissen von 7.9 und 4.5 erhalten. Die Wahl der Hilfsbase stellt eine weitere experimentelle Variable dar, die für die Katalysatoren **5** und **11** untersucht wurde. Es stellt sich dabei überraschenderweise heraus, dass die katalytische Effizienz bei Einsatz der stärkeren Hilfsbase EtN(iPr)$_2$ (Hünig-Base) im Vergleich zu NEt$_3$ für beide Katalysatoren vergleichbar absinkt.

Alkyl-substituierte 3,4-Diaminopyridine stellen eine neue Klasse von Katalysatoren für die Acylierung von Alkoholen dar. Die katalytische Effizienz dieser Verbindungen kann innerhalb einer einzigen synthetischen Strategie durch Variation der Stickstoff-Substituenten in breitem Maße verändert werden. Die katalytische Aktivität der besten dieser Katalysatoren ist vergleichbar zu den besten bisher bekannten carbocyklischen Derivaten von DMAP. Im Gegensatz zu diesen letzteren Verbindungen sind die aktivsten 3,4-Diaminopyridin-Katalysatoren durch eine flexible 3-oder 4-stufige Synthese einfach zugänglich, die für weitere strukturelle Variationen leicht modifiziert werden kann.

**Experimenteller Teil**

Geometrie-Optimierungen wurden mit dem Becke3LYP Hybrid-Funktional in Kombination mit dem *split valence* Basissatz 6-31 G(d) durchgeführt. Thermochemische Enthalpiekorrektur bei einer Temperatur von 298.15 K wird durch eine Frequenzrechnung auf demselben Niveau durchgeführt. Singlepoint Rechnungen werden auf dem Becke3LYP/6-311+G(d,p)-Niveau durchgeführt. Eine Kombination der erhaltenen Energie mit der thermochemischen Korrektur auf dem Becke3LYP/6-31G(d)-Niveau ergibt die $H_{298}$-Werte, die im Text beschrieben werden. Alle Rechnungen werden mit Gaussian 03 durchgeführt.[21]

Alle Reaktionen die unter Feuchtigkeitsausschluss durchgeführt worden sind, werden unter Stickstoffatmosphäre in trockenen Lösungsmitteln und durch Trockenschrank und Heißluftfön getrockneten Reaktionsapparaturen durchgeführt. THF wird unter Stickstoffatmosphäre von Natriumhydrid destilliert. Methyl-tert.-butylether (MTBE), Pyridin,

Triethylamin und Deuterochloroform werden unter Stickstoffatmosphäre von Calciumhydrid destilliert. Acetanhydrid und Isobuttersäureanhydrid werden unter vermindertem Druck von $P_4O_{10}$ auf trockenes $K_2CO_3$ destilliert, filtriert und im Vakuum fraktioniert. Beide Anhydride werden über 4 Å-Molsieb unter Stickstoffatmosphäre aufbewahrt. Ethanol, Dichlormethan, Ethylacetat und Methanol wird am Rotationsverdampfer destilliert. *n*-BuLi wird mit Diphenylessigsäure titriert und die Molarität bestimmt. Alle anderen Reagenzien, wenn nicht anders im Text erwähnt, werden mit der höchst möglichen Qualität ohne weitere Aufreinigung verwendet. Dünnschichtchromatographie wird auf mit Fluoreszenzfarbstoff markierten DC-Platten der Firma Merck KGaA (Silikagel 60 $F_{254}$, Schichtdicke 0.2 mm) und auf Fluka (basisch Aluminiumoxid $F_{254}$, Brockman-Aktivität 1, pH 9.5, Schichtdicke 0.2 mm) durchgeführt. Flash-Chromatographie wird mit Merck KGaA Silikagel 60 (Korngröße 0.040-0.063 mm) und Fluka basisches Aluminiumoxid (basisch Aluminiumoxid $F_{254}$, Brockman-Aktivität 1, pH 9.5, Körngröße 0.05-0.15 mm) bei einem Druck von 1.5 bar für Silikagel und 0.5 bar für basisches Aluminiumoxid durchgeführt. $^1$H- und $^{13}$C-NMR Spektren werden mit einem Varian Mercury 200, Varian 300, Varian INOVA 400 und Varian 600 Gerät aufgenommen. NOE-Spektren werden in $CDCl_3$ bei 27 °C aufgenommen. Chemische Verschiebungen werden in ppm relativ zum Lösungsmittelpeak angegeben.[22] Folgende Abkürzungen werden verwendet um die Multiplizitäten der Signal im $^1$H-NMR-Spektrum zu charakterisieren: s=Singulett, d=Dublett, t=Triplett, q=Quartett, quin=Quintett, sex=Sechstett, sep=Septett, m=Multiplett, b=breit und Kombinationen der verwendeten Abkürzungen. Alle $^1$H- und $^{13}$C- Signale werden mit COSY-, NOESY-, HSQC-, HMBC- und DEPT-Experimenten zugeordnet. $^1$H-NMR-Spektren der Kinetikmessungen werden mit dem Programm VNMR 4.3 Rev. G0194 ausgewertet. Integrale der relevanten Peaks werden automatisch mit einem selbst geschriebenen Unterprogramm ausgewertet. Die Subroutine wird mit MAGICAL™ II-Programming geschrieben. IR-Spektren werden mit KBr-Presslingen als Probenmedium mit einem Perkin-Elmer 1420 Infrarot-Spektrometer und einem Perkin-Elmer FT-IR Spectrum BX Spektrometer unter Verwendung der ATR-Technik aufgenommen. Alle Signale sind als vs=sehr intensiv, s=intensiv, m=mittel und w=schwach aufgeführt. Massenspektren sind mit einem Finnigan MAT 95 unter Verwendung von Elektronenstoß-Ionisation (EI, 70 eV) oder chemischer Ionisation (CI, Isobutan) als Ionisationsquelle aufgenommen. ESI-MS Spektren werden mit einem Thermo Finnigan LTQ FT-Instrument aufgenommen. Gaschromatogramme werden mit einem Varian 3400 GC mit einer 25 m CS-Supreme-5 Kapillarsäule und einem Finnigan MAT 95 Massenspektrometer als Detektor aufgezeichnet. Im Falle der elektronenreichen Pyridine **11, 5, 6, 20** und **32** wird das Lösungsmittel nach Flashchromatographie durch Einleiten eines Stickstoffstroms in den Rotationsverdampfer abdestilliert.

**Allgemeine Durchführung zu den Reaktivitätsexperimenten A und B.**

Deutorochloroform, Triethylamin und Hünigbase werden vor der Verwendung frisch unter Stickstoffatmosphäre von Calciumhydrid destilliert. Alle kinetischen Messungen werden bei konstanter Temperatur bei 23 °C für Reaktion **A** und 40 °C für Reaktion **B** auf einem Varian Mercury 200 Spektrometer aufgenommen. In drei trockenen 5 ml Messkolben werden folgende Maßlösungen in deuteriertem Chloroform hergestellt.

A: 1.2 M Acetanhydrid-Lösung.
B: 0.6 M Ethinylcyclohexanol-Lösung und 1.8 M Triethylamin- oder Hünigbasen-Lösung.
C: 0.06 M Katalysatorlösung.

**Probenvorbereitung und Kinetikmessung für Reaktion A.** In einem NMR-Rörchen werden jeweils 200 µl der oben genannten Maßlösungen mittels einer Eppendorf-Pipette pipettiert. Die Reaktionsmischung wird vermischt und sofort in das NMR-Spektrometer eingeführt. Die Reaktion wird durch Aufnahmen von NMR-Spektren in einem definierten Zeitintervall bis zum 100%igen Umsatz verfolgt.

**Probenvorbereitung und Kinetikmessung für Reaktion B.** Durchführung wie unter Reaktion **A** beschrieben nur das die NMR-Röhrchen, um ein Verdampfen des Lösungsmittels zu verhindern, mit einem Brenner zu geschmolzen worden sind.

**Bestimmung der Halbwertszeit für Reaktion A.** Alle Signal die den Protonen einer AcetylGruppen zu geordnet werden können werden nach der Aufnahme der Spektren automatisch integriert. Dabei wird Acetanhydrid, Ester und Triethylammoniumacetat mit folgenden Integralgrenzen integriert: $\pm 8$, $\pm 2$ und $\pm 6$ Hz. Der Umsatz wird mit Gleichung **2** berechnet. Der Umsatz wird als Funktion der Zeit mit Gleichung **4** bis zur Selbstkonsistenz gefittet. Die Halbwertszeit wird mit Funktion **4** bei 50%igen Umsatz berechnet.

**Bestimmung der Halbwertszeit für Reaktion B.** Das Dublett der Isobuttersäuregruppe als auch das Signal von Dioxan werden nach Aufnahme der NMR-Spektren automatisch integriert. Dabei wird Dioxan und Isobuttersäureanhydrid mit den gleichen Integralgrenze von $\pm 2$ Hz integriert. Der Umsatz wird mit Gleichung **3** berechnet. Der Umsatz wird als Funktion der Zeit mit Gleichung **4** bis zur Selbstkonsistenz gefittet. Die Halbwertszeit wird mit Funktion **4** bei 50%igen Umsatz berechnet.

$$Conv. = \left( \frac{I_{Ester}}{1/4\left(I_{Ac_2O} + I_{Ester} + I_{HNEt_3OAc}\right)} \right) * 100\% \qquad \text{Eq. 2}$$

$$Conv. = \left( 1 - \frac{\left(I_{Anhydride}\right)}{3\left(I_{Dioxane}\right)} \right) * 200\% \qquad \text{Eq. 3}$$

$$y = A\exp\left( -\frac{\left(x - x_0\right)}{t_0} \right) + const. \qquad \text{Eq. 4}$$

**Arbeitsvorschriften**

**Pyrido[3,4-b]pyrazine (23):** Zu einer Lösung von 2.82 ml Glyoxal (40 wt. % in Wasser, ρ=1.265 g/ml, 62.48 mmol) in 30 ml Ethanol gibt man 2.00 g (18.33 mmol) 3,4-Diaminopyridin. Die Reaktionsmischung wird für 5 h bei 70 °C Ölbadtemperatur gehalten und nachdem abkühlen auf Raumtemperatur wird das Lösungsmittel am Rotationsverdampfer abdestilliert. Das Rohprodukt wird durch Flashchromatographie (EtOAc/Isohexan, 9:1) gereinigt. Man erhält 2.35 g (17.96 mmol, 98%) eines weißen Pulvers. $R_f$=0.27 (Isohexan/FtOAc, 1:9). $^1$H NMR (200 MHz, $CDCl_3$): δ=7.94 (dd, $^3J$=5.8 Hz, $^4J$=0.6 Hz, 1H, H-7), 8.83 (d, $^3J$=5.8 Hz, 1H, H-8), 8.96 (d, $^3J$=1.6 Hz, 1H, H-3), 9.02 (d, $^3J$=1.6 Hz, 1H, H-2), 9.56 (d, $^4J$=0.6 Hz, 1H, H-5) ppm. $^{13}$C NMR (100 MHz, $CDCl_3$): δ=121.6 (CH, C-7), 138.0 (Cq), 145.3 (Cq), 147.3 (C-H, C-8), 146.5 (C-H, C-3), 149.2 (CH, C-2), 154.8 (C-H, C-5) ppm. GC-MS (EI): RT 5.45 min, *m/z* (%): 132 (8), 131 ($M^+$, 100), 104 (25), 77 (13), 50 (10) IR (KBr): □ = 3435 (vs), 3092 (w), 3023 (m), 1969 (w), 1758 (w), 1631 (w), 1598 (vs), 1562 (m), 1536 (w), 1488 (s), 1436 (vs), 1416 (m), 1381 (m), 1351 (w), 1290 (w), 1279 (m), 1212 (m), 1201 (m), 1148 (w), 1033 (s), 1014 (s), 972 (w), 959 (vs), 931 (m), 881 (vs), 838 (m), 824 (s), 773 (w), 651 (s), 623 (m), 546 (w), 524 (w), 457 (s) $cm^{-1}$.

**1,2,3,4-Tetrahydropyrido[3,4-b]pyrazin (26):** Zu einer Lösung von Chinoxalin **23** (2.90 g, 0.022 mol) in 100 ml trockenen Ethanol gibt man 2.90 g (0.076 mol) $NaBH_4$-Pulver. Man erhitzt auf 40 °C Ölbadtemperatur und hält die Reaktionslösung für 24 h bei dieser Temperatur. Anschließend wird die Reaktionslösung auf Raumtemperatur abgekühlt und 3 ml Wasser zugegeben. Der anorganische Feststoff wird abgesaugt zweimal mit 20 ml DCM gewaschen. Die vereinigten organischen Phasen werden über Natriumsulfat getrocknet und das Lösungsmittel am Rotationsverdampfer abdestilliert. Das erhaltene Rohprodukt wird durch Flashchromatographie auf basischen Aluminiumoxid (EtOAc/MeOH, 10:1) gereinigt. Man erhält einen weißen Schaum. $R_f$ = 0.15 (basisches Alox, 20:1, DCM:MeOH). $^1$H NMR:(200 MHz, $CDCl_3$): δ = 3.37 (d, $^3J$= 5.8 Hz, 1H, H-3), 3.38 (d, $^3J$= 5.8 Hz, 1H, H-2), 6.29 (d, $^3J$= 5.4 Hz, 1H, H-8), 7.67 (d, $^3J$= 5.4 Hz, 1H, H-7), 7.67 (s, 1H, H-5). $^{13}$C NMR: (100 MHz, $CDCl_3$): δ = 40.1 ($CH_2$), 41.0 ($CH_2$), 107.8 (C5), 129.6 (Cq), 135.3 (C-H, C7), 140.1 (Cq), 141.1 (C-H, C-5). GC-MS RT 8.09 min (EI) *m/z* (%): 136 (8), 135 ($M^+$ 79), 134 ($M^+$-$H^+$,100), 133 (10), 132 (7), 131 (2), 120 (4), 107 (7), 105 (4), 94 (2), 93 (7), 80 (2), 79 (3), 78 (4), 67 (4), 66 (2), 53 (2), 52 (2), 52 (3), 51 (2). IR (KBr): □ = 3349 (m), 2859 (m), 1593 (s), 1534 (s), 1474 (s), 1344 (s), 1311 (s), 1281 (s), 1256 (w) 1228 (m), 1182 (m), 1102 (m), 1051 (w), 1038 (m), 893 (m), 862 (m), 824 (s), 772 (m) $cm^{-1}$. HRMS (EI) (%) berechnet für $C_7H_8N_3$ (M-$H^+$): 134.0718 gefunden: 134.0709.

**1-(4-Acetyl-3,4-düiydro-2H-pyrido[3,4-b]pyrazin-1-yl)-ethanon (8):** Zu einer 0°C warmen Lösung von Tetrahydrochinoxalin **26** (1.49 g, 0.011 mol) in 60 ml Pyridin werden 23 ml (24.71 g, 24.20 mol) Acetanhydrid zugegeben. Die Reaktionsmischung wird anschließend auf 100 °C für 48 h erhitzt. Nach dem abkühlen auf Raumtemperatur wird das Lösungsmittel im Vakuum abdestilliert und der gelbe Feststoff durch Flashchromatographie (EtOAc:MeOH, 10:3) gereinigt. Man erhält 1.93 g (8.80 mmol, 80 %) eines schwach gelben Feststoffs. $^1$H NMR (400 MHz, $CDCl_3$): δ = 2.25 (s, 3H, $CH_3$), 2.31 (s, 3H, $CH_3$), 3.90 (ddd, $^2J$= 12 Hz, $^3J$= 4 Hz, $^3J$=4 Hz, 2H, $CH_2$), 3.94 (ddd, $^2J$ = 12 Hz, $^3J$ = 4 Hz, $^3J$= 4 Hz, 2H, $CH_2$), 7.67 (s, 1H, H-5), 7.87 (bs, 1H, H-5), 8.32 (d, $^3J$ = 4 Hz, 1H, H-7), 8.46 (m, 1H, H-8). $^{13}$C NMR (100 MHz, $CDCl_3$): δ = 22.3, 22.8 ($CH_3$), 42.1, 46.5 ($CH_2$), 117.4 (C5), 128.2 ($C_q$), 139.0 (Cq), 145.7 (C-7), 146.9 (C-8), 168.6 (C=O). GC-MS(EI) RT 9.66 min, *m/z* (%): 220 (14), 219 ($M^+$, 81), 178 (11), 177 ($M^+$-AcO,100), 176 (10), 162 (3), 159 (2), 136 (7), 135 (78), 134 ($M^+$-2AcO,99), 133 (8), 132 (9), 120 (4), 119 (2), 107 (5), 105 (2), 93 (3), 80 (2), 79 (4), 78 (4), 52 (2), 51 (2), 43 ($AcO^+$,20); IR (neat): □ = 2960 (w), 1684 (s) 1654 (vs) 1582 (m) 1494 (s), 1407 (vs), 1330 (m), 1320 (vs), 1277 (m), 1259 (s), 1248 (m), 1217 (s), 1248 (s), 1217 (s), 1179 (m), 1150 (w), 1118 (m), 1064 (m), 1033 (s),

969 (s), 886 (w), 857 (m), 846 (s), 799 (s), 764 (w), 749 (w), 704 (w) $cm^{-1}$. HRMS (EI) berechnet für $C_{11}H_{13}N_3O_2$ [$M^+$]: 219.1008; gefunden: 219.0995.

**1,4-Diethyl-1,2,3,4-tetrahydropyrido[3,4-b]pyrazin (20):** In 60 ml MTBE suspendiert man 1.56 g (11.69 mmol) $AlCl_3$ bei Raumtemperatur. Nach 45 min rühren wird die Reaktionsmischung auf 0 °C gekühlt und Portionsweise (1.32 g, 34.65 mmol) $LiXlH_4$ zugegeben. Nach beendeter Zugabe und weiteren rühren für 15 min wird Verbindung **8** (1.00 g, 4.56 mmol) zugegeben und für eine weitere Stunde bei 0 °C gerührt. Anschließend wird für 8 h unter Rückfluß erhitzt und nachdem abkühlen auf Raumtemperatur die Reaktionsmischung auf Eiswasser gegossen. Der ausgefallene anorganische Feststoff wird abgesaugt und zweimal mit 30 ml DCM gewaschen. Die Wasserphase wird auf pH 12 gebracht und dreimal mit 40 ml DCM extrahiert. Die vereinigten Extrakte werden über $Na_2SO_4$ getrocknet und das Lösungsmittel am Rotationsverdampfer abdestilliert. Das Rohprodukt wird durch Flashchromatographie an Kieselgel gereinigt ($EtOAc/MeOH/NEt_3$, 10:0.5:1). Man erhält 0.52 g (60%) eines farblosen Öls, was im Gefrierschrank fest wird. $R_f$ = 0.47 ($EtOAc/MeOH/NEt_3$, 10:0.5:1). $^1H$ NMR: (400 MHz, $CDCl_3$): δ = 1.15 (q, $^3J$ = 14 Hz , 6H, $CH_3$), 3.22 (m, $^3J$ = 6 Hz, 2H, H-3), 3.23 (m, $^2J$ = 14 Hz, 4H, $CH_2$), 3.42 (m, $^3J$= 6 Hz, 2H, H-2), 6.34 (d, $^3J$= 5.2 Hz, 1H, H-8), 7.69 (s, 1H, H-5), 7.75 (d, $^3J$= 5.2 Hz, 1H, H-7). $^{13}C$ NMR: (100 MHz, $CDCl_3$): δ = 10.1, 10.4 ($CH_3$), 44.6, 46.5 ($CH_2$, C-2, C-3), 44.9, 45.0 ($CH_2$), 104.0 (C-8), 130.5, 130.1 (Cq), 131.8 (C-5), 140.7 (C-7). GC-MS(EI): RT 8.63 min, *m/z* (%): 192 (10), 191 ($M^+$,100), 190 (4), 177 (8), 176 (95), 175 (5), 162 (11), 161 (8), 160 (5), 148 (13), 147 (10), 146 (8), 135 (2), 134 (9), 133 (6), 131 (8), 121 (2), 119 (4), 118 (3), 107 (3), 104 (2), 92 (2), 91 (2), 80 (8), 77 (4) IR (KBr): □ = 3436 (s), 3112 (w), 3019 (w), 2964 (w), 1661 (s), 1686 (vs), 1583 (m), 1497 (m), 1409 (s), 1363 (w), 1332 (m), 1311 (s), 1260 (m), 1249 (w), 1232 (m), 1220 (m), 1180 (m), 1150 (w), 1119 (m), 1064 (w), 1035 (m), 985 (m), 970 (m), 886 (s), 858 (m), 847 (m), 802 (m), 765 (w), 740 (w), 704 (w), 647 (w), 614 (w), 592 (w), 577(m), 570 (m), 507 (w) $cm^{-1}$. HRMS (EI) berechnet für $C_{11}H_{17}N_3$ [$M^+$]: 191.1422; gefunden: 191.1430.

**1,4-Dimethy-1,2,3,4-tetrahydropyrido[3,4-b]pyrazin (6):** Unter Eiskühlung gibt man zu Tetrahydrochinoxalin 26 (1.26 g, 9.32 mmol) 35 ml Ameisensäure. Anschließend gibt man 12 ml Formaldehyd-Lösung hinzu (<37 % in Wasser) und man erhitzt für 48 h auf 110 °C Ölbadtemperatur. Anschließend läßt man auf Raumtemperatur abkühlen gibt unter Eiskühlung ca. 150 ml 20%ige Natronlauge hinzu. Dabei sollte der pH-Wert nicht 12 überschreiten. Die Mutterlauge wird mit 250 ml DCM in einem Flüssig/Flüssig-Extraktor über Nacht extrahiert. Die organische Phase wird über $Na_2SO_4$ getrocknet und das Lösungsmittel am Rotationsverdampfer abdestilliert. Das Rohprodukt wird durch Flashchromatographie an Kieselgel ($EtOAc/MeOH/NEt_3$, 10:1:1) und basischen Aluminiumoxid (EtOAc/MeOH, 10:1) gereinigt. Man erhält 0.849 g (5.32 mmol, 57%) eines gelben Feststoffs. $R_f$ = 0.56 (basic alumina, EtOAc/MeOH, 10:1). $^1H$ NMR: (400 MHz, $CDCl_3$): δ = 2.77 (s, 3H, $CH_3$), 2.82 (s, 3H, $CH_3$), 3.12 (m, 2H, $CH_2$-B), 3.37 (m, 2H, $CH_2$-C), 6.21 (d, $^3J$= 5.6 Hz, 1H, H-7), 7.56 (s, 1H, H-5), 7.74 (d, $^3J$= 5.6 Hz, 1H, H-8) ppm. $^{13}C$ NMR: δ = (75 MHz,$CDCl_3$): 38.0 (N-1-$CH_3$), 39.1 (N-4-$CH_3$), 48.8 (C-3), 49.6 (C-2), 104.0 (C-7), 131.2 (C-5), 132.3 (C-4a), 141.6 (C-7), 142.2 (C-8a) ppm; GC-MS(EI): RT 8.05 min, *m/z* (%): 164 (8), 163 ($M^+$,100), 162 (21), 161 (5), 149 (4), 148 (40) 147 (9), 146 (8), 134 (8), 133 (11), 132 (6), 121 (4), 120 (2), 119 (6), 107 (3), 105 (2), 93 (2), 92 (4), 81 (6), 80 (4), 79 (2), 78 (4), 66 (3), 51 (2), 42 (4). IR (neat): □ = 3378 (w), 3035 (w), 2979 (w), 2873 (m), 2826 (s), 2792 (w), 1581 (s), 1519 (s), 1466 (s), 1454 (s), 1435 (m), 1416 (m), 1380 (w), 1335 (vs), 1290 (s), 1250 (w), 1235 (vs), 1214 (m), 1172 (s), 1114 (s), 1099 (s), 1069 (s), 1030 (m), 936 (w), 911 (w), 883 (s), 815 (s), 800 (s), 783 (s), 746 (m), 709 (m), 622 (m) $cm^{-1}$. HRMS (EI) berechnet für $C_9H_{13}N_3$ [$M^+$]: 163.1109; gefunden: 163.1089.

**6,7,8,9-Tetrahydropyrido[3,4-*b*]chinoxalin (24):** Zu einer Suspension von 1.56 g (13.93 mmol) 3,4-Diaminopyridin in 50 ml Ethanol gibt man (1.52 g, 13.93 mmol) 1,2-Cyclohexandion. Anschließend erhitzt man die Reaktionsmischung für 5 h auf 70 °C Ölbadtemperatur, kühlt danach auf Raumtemperatur ab und destilliert danach das Lösungsmittel am Rotationsverdampfer ab. Das erhaltene Rohprodukt wird durch Flashchromatoprahie mit Ethylacetat als Eluent gereinigt. Man erhält 1.84 g (71 %) eines weißen Feststoffs, der im Kühlschrank aufbewahrt und nach 1-2 Tagen weiterverarbeitet werden sollte. Durch stehen lassen im offenen Kolben im Sonnenlicht verändert sich die Farbe des Feststoff nach grau-grün. $R_f$=0.25 (EtOAc/Hexan, 20:1). $^1H$ NMR:(200 MHz, $CDCl_3$): δ = 2.05 (m, 4H, H-7,8), 3.18 (m, 4H, H-6,9), 7.79 (d, $^3J$= 5.8 Hz, 1H), 8.72 (d, $^3J$ = 5.8 Hz, 1H), 9.38 (s, 1H) ppm. $^{13}C$ NMR (75 MHz, $CDCl_3$): δ = 22.8 ($CH_2$, C-7,8), 33.7 ($CH_2$, C-6,9), 121.2 (CH), 136.9 (Cq), 144.1 (Cq), 146.8 (CH), 153.9 (CH), 156.8 (Cq) 159.9 (Cq) ppm. GC-MS(EI): RT 8.44 min, *m/z* (%) 186 (11), 185 ($M^+$,100), 184 (39), 183 (3), 182 (3), 171 (2), 170 (21), 169 (4), 158 (2), 157 (5), 156 (5) 131 (2), 104 (2), 103 (4), 78 (3), 76 (4), 67 (2), 64 (2), 51 (2), 50 (6); IR (KBr): □ = 3435 (vs), 2947 (s), 2864 (m), 1594 (s), 1557 (w), 1461 (w), 1421 (m), 1385 (s), 1365 (w), 1330 (w), 1297 (m), 1211 (m), 1140 (w), 979 (m), 949 (w), 901 (m), 849 (m), 679 (w), 629 (w), 570 (w), 412 (w) $cm^{-1}$. HRMS (EI): berechnet für $C_{11}H_{11}N_3$ 185.0953 [$M^+$], gefunden: 185.0935

**5,5a,6,7,8,9a,10-Octahydro-pyrido[3,4-*b*]chinoxalin (27):** In einen 250 ml Schlenkkolben gibt man 6.13 g (33.09 mmol) **24** und 100 ml THF. Man kühlt die Lösung auf -40 °C und gibt 3.09 g (81.42 mmol, 2.5 eq.) $LiAlH_4$ in kleinen Portionen hinzu. Die Reaktionsmischung wird auf Raumtemperatur aufgewärmt und 32 h bei Raumtemperatur gerührt. Anschließend gießt man auf Eiswasser und stellt die Wasserphase auf ein pH-Wert von 12 ein. Der anorganische Feststoff wird abgetrennt und zweimal mit 40 ml DCM gewaschen. Die Mutterlauge wird 8 h mit 250 ml DCM in einem Flüssig/Flüssig-Extraktor extrahiert. Die vereinigten organischen Phasen werden über $Na_2SO_4$ getrocknet und das Lö-

sungsmittel am Rotationsverdampfer abdestillert. Flashchromatographie ($EtOAc/MeOH/NEt_3$, 10:1:1) des erhaltenen Rohprodukts liefert 5.63 g (29.78 mmol, 90%) eines weißen Feststoffs. $R_f$=0.36 ($EtOAc/MeOH/NEt_3$, 10:1:1). $^1H$ NMR: (300 MHz, $CDCl_3$): δ = 1.32-1.40 (m, 3H), 1.56-1.75 (m, 5H), 3.41 (m, 1H), 3.46 (s, 1H, N-H), 3.51 (m, 1H), 4.20 (s, 1H, N-H), 6.29 (d, 1H, $^3J$ = 5.4 Hz, H-3), 7.65 (d, 1H, $^3J$ = 5.4 Hz, H-4), 7.68 (s, 1H, H-1) ppm. $^{13}C$ NMR (75 MHz, $CDCl_3$): δ = 22.0, 22.2 (C-7,8), 30.4, 30.8 ($CH_2$, C-6,9), 49.2 (CH, C-5a), 50.1 (CH, C-9a), 107.7 (C-4), 128.8 (Cq, C-10a), 134.8 (C-3), 139.2 (Cq, C-4a) 140.7 (C-4) ppm. GC-MS (EI): RT 10.06 min, *m/z* (%) 190 (13), 189 ($M^+$, 99), 188 (9), 187 (3), 185 (10), 184 (4), 170 (4), 160 (10), 159 (3), 158 (5), 148 (2) 147 (16), 146 (100), 145 (2), 134 (13), 133 (17), 132 (15), 120 (10), 119 (3), 105 (2) 94 (3), 93 (2), 78 (3), 66 (2), 40 (2); IR (neat):□ = 3220 (s), 2927 (s), 2852 (s), 2354 (m), 1725 (w), 1595 (vs), 1523 (vs), 1456 (w), 1443 (m), 1403 (w), 1362 (s), 1294 (s), 1269 (s), 1240 (m), 1206 (s), 1175 (s), 1087 (m), 1052 (m), 1003 (m), 938 (m), 884 (m) 810 (vs) 725 (m) $cm^{-1}$. HRMS (EI): berechnet für $C_{11}H_{15}N_3$ 189.1266 [$M^+$], gefunden: 189.1259.

Die Reaktion kann alternativ auch nach der Vorschrift von Opatz et al. mit $NaBH_4/BH_3/H_2NCH_2CH_2OH$ als Reagenz durchgeführt werden, wobei eine Ausbeute von 74 % erzielt wird.[16]

**1-(10-Acetyl-6,7,8,9,9a,10-hexahydro-5a*H*-pyrido[3,4-*b*]chinoxalin-5-yl)-ethanon (14):** In einen 250 ml Rundkolben gibt man unter Eiskühlung **27** (1.20 g, 6.34 mmol), 40 ml Pyridin, 30 ml (32.36 g, 317 mmol, p=1.082 g/ml) Acetanhydrid und 0.234 g (25 mol%) PPY. Die Reaktionmischung wird auf 100 °C Ölbadtemperatur erhitzt und 48 h bei dieser temperatur gehalten. Durch DC-Kontrolle auf basischen Aluminiumoxid (EtOAC/MeOH, 10:1) kann die Reaktion verfolgt werden. Nach dem abkühlen auf Raumtemperatur wird das Lösungsmittel unter verminderten Druck abdestilliert und des erhaltene Rohprodukt durch Flashchromatographie auf basischen Aluminiumoxid (EtOAc/MeOH, 10:1) gereinigt. Man erhält einen dunkelgelben Feststoff (1.17 g, 4.31 mmol, 68 %). $R_f$= 0.45 (EtOAc/MeOH, 10:1). $^1H$ NMR:(400 MHz, $CDCl_3$): δ = 1.34 (m, 4H), 1.60 (d, 4H), 2.23 (s, 3H, $H_3C$-CO-N), 2.27 (s, 3H, $H_3C$-CO-N), 4.74 (m, 1H), 4.85 (m, 1H), 7.25 (d, $^3J$= 5.6 Hz, 1H, H-4), 8.41 (d, $^3J$= 5.6 Hz, H-3), 8.49 (s, 1H, H-1) ppm. $^{13}C$ NMR (75 MHz, $CDCl_3$): δ = 21.7, 21.9 (C-7,8), 23.1, 23.6 ($H_3C$-CO-N), 28.4, 28.5 (C-6,9), 55.2, 56.0 (*C*H, C-5a,9a), 119.6 (C-4), 130.8 (Cq, C-10a), 141.6 (Cq, C-4a), 147.1, 147.2 (C-1, C-3) 169.1, 169.2 (C=O) ppm. GC-MS(EI): RT 10.45 min, *m/z* (%) 274 (13), 273 ($M^+$, 58), 232 (15), 231 (100), 230 (42), 216 (5), 214 (5), 213 (5), 203 (3), 202 (4), 190 (13), 189 (84), 188 (45), 173 (5), 172 (6), 160 (7), 159 (5), 158 (5), 147 (8), 146 (40) 134 (6), 133 (13), 132 (15), 120 (7), 43 (9); IR (neat): □ = 2940 (m), 1660 (vs), 1587 (m), 1559 (w), 1498 (s), 1448 (w), 1427 (w), 1388 (m), 1353 (w), 1337 (m), 1289 (s), 1270 (s), 1248 (s), 1248 (m), 1220 (w), 1183 (w), 1102 (w), 1036 (m), 978 (m), 857 (w), 839 (w) 777 (w) $cm^{-1}$. HRMS (EI): berechnet für $C_{11}H_{15}N_3$ 273.1477 [$M^+$], gefunden: 273.1482.

**5,10-Diethyl-5,5a,6,7,8,9,9a,10-octahydro-pyndo[3,4-*b*]chinoxalim (11):** Zu einer Suspension von 0.77 g (5.80 mmol) $AlCl_3$ in 30 ml MTBE gibt man nach 45 min rühren bei Raumtemperatur und anschließendem kühlen auf 0 °C 1.32 g (9,35 mmol) $LiAlH_4$ in kleinen Portionen. Nach beendeter Zugabe und weiterem rühren für 15 min gibt man Verbindung **14** (0.64 g, 2.23 mmol) hinzu und läßt 1 h bei 0 °C rühren. Anschließend wird für 8 h auf Rückfluß erhitzt und dann nach abkühlen auf Raumtemperatur auf Eiswasser gegossen. Der anorganische Niederschlag wird abgesaugt und zweimal mit 30 ml DCM gewaschen. Die Wasserphase wird auf pH 12 gebracht und dreimal mit 40 ml DCM extrahiert. Die kombinierten organischen Phasen werden über Na2SO4 getraocknet und das Lösungmittel am Rotationsverdampfer abdestilliert. Das erhaltene Rohprodukt wird durch Flashchromatographie an Kieselgel ($EtOAc/MeOH/NEt_3$, 10:2:1) und an basischen Aluminiumoxid (EtOAc/MeOH, 10:1) gereinigt. Man erhält 0.30 g (1.22 mmol, 55 %) eines farblosen Feststoffs. $R_f$=0.32 (basic alumina, EtOAc/MeOH, 10:1). $^1H$ NMR:(400 MHz, $CDCl_3$): δ = 1.11, 1.13 (t, $^3J$ = 7.2 Hz, $H_3C$-$CH_2$-N-5, $^3J$ = 7.2 Hz, $H_3C$-$CH_2$-N-10, 6-H), 1.37 (m, 2H, H-7,8), 1.56 (m, $^3J_{aa}$ = 6.8 Hz, $^3J_{ee}$ = 3.2 Hz, 4H, H-6, H-9, H-7, H-8), 1.76 (m, $^3J_{ae}$ = 3.2 Hz, 1H, H-9), 1.89 (m, $^3J_{ae}$ = 3.2 Hz, 1H), 3.18 (dq, $^3J$ = 7.2 Hz, $^2J$ = 14.4 Hz, 2-H, $H_3C$-$CH_{AB}$-N-5, $H_3C$-$CH_{AB}$-N-10), 3.23 (ddd, $^3J_{ae}$ = 2.8 Hz, $^3J_{ae}$, = 3.2 Hz, $^3J_{aa}$ = 6.8 Hz, 1H, H-5a), 3.34 (ddd, $^3J_{ae}$ = 2.8 Hz, $^3J_{ae}$ = 3.2 Hz, $^3J_{ae}$ = 3.2 Hz, H-1, H-9a), 3.43 (dq, $^3J$ = 7.2 Hz, $^2J$ = 14.4 Hz, $H_3C$-$CH_{AB}$-N-5, $H_3C$-$CH_{AB}$-N-10, 2H, H-1), 6.33 (d, $^3J$ = 5.6 Hz, 1H, H-4), 7.67 (s, 1H, H-1), 7.72 (d, 1H, H-3) ppm. $^{13}C$ NMR (100 MHz, $CDCl_3$): δ = 10.8 ($H_3C$-$CH_2$-N-5), 11.8 ($H_3C$-$CH_2$-N-10), 21.7 (C-7), 22.9 (C-8), 27.3 (C-6), 27.8 (C-9), 40.4 ($H_3C$-$CH_2$-N-5), 41.7 ($H_3C$-$CH_2$-N-10), 52.8 (C-9a), 56.4 (C-5a), 104.3 (C-4), 130.6 (C-10a, C-1), 139.4 (C-3), 141.2 (C-4a) ppm. GC-MS(EI): RT 10.58 min, *m/z* (%) 246 (18), 245 ($M^+$, 100), 231 (9), 230 (57), 217 (7), 216 (44), 207 (6), 186 (7), 174 (15), 162 (6), 160 (16), 158 (6), 148 (12), 146 (6), 132 (8); IR (neat): □ = 3436 (s), 2970 (m), 2860 (vs), 2860 (s), 1628 (w), 1576 (vs), 1513 (vs), 1473 (w), 1447 (m), 1348 (s), 1317 (w), 1267 (s), 1211 (s), 1167 (w), 1125 (w), 1107 (w), 1077 (w), 1059 (w), 1040 (w), 920 (w), 798 (s) 777 (m), 746 (m) $cm^{-1}$. HRMS (EI): berechnet für $C_{15}H_{23}N_3$ 245.1892 [$M^+$], gefunden: 245.1889.

**1-(6,7,8,9,9a,10-Hexahydro-5a*H*-pyrido[3,4-*b*]chinoxalin-5-yl)-ethanon (28):** In einem 100 ml Schlenkkolben gibt man **27** (1.0 g, 5.28 mmol), 20 ml DCM, 2.20 ml (15.84 mmol) $NEt_3$ und 5 mol% PPY (39 mg, 0.264 mmol). Anschließend gibt man 0.54 ml (5.81 mmol) Acetanhydrid hinzu und rührt die Reaktionsmischung für 30 min. Anschließend stoppt man die Reaktion durch Zugabe von 2 ml MeOH und rührt für weitere 10 min. Man destilliert das Lösungsmittel am Rotationsverdampfer ab und reinigt das erhaltene Rohprodukt durch Flashchromatographie ($EtOAc/MeOH/NEt_3$, 10:1:1). Man erhält 1 g (4.31 mmol, 82 %) von **28.** Das erhaltene Produkt enthält Verunreinigungen von PPY und wird ohne weitere Reinigung weiter umgesetzt. $R_f$=0.44 ($EtOAc/NEt_3/MeOH$, 10:1:1). $^1H$ NMR:(300 MHz, $CDCl_3$): δ = 1.44 (m, 5H), 1.76 (m, 3H), 2.29 (s, 3H, $H_3C$-CO-N), 3.59 (m, 1H), 4.07 (m, 1H), 7.08 (bs, 1H, H-4), 7.85 (d, $^3J$= 5.4 Hz, 1H,

H-3), 7.98 (s, H-1, H-1). $^{13}C$ NMR (75 MHz, $CDCl_3$): δ = 18.5 ($CH_2$), 23.5 ($H_3C$-CO-N), 24.8, 25.4 ($CH_2$), 31.08 ($CH_2$), 48.9 (C-9a), 118.1 (Cq, C-4a), 127.9 (Cq, C-10a), 132.2 (C-4), 136.6 (C-1), 138.0 (C-3), 169.0 (C=O) ppm HRMS (EI): berechnet für $C_{13}H_{17}N_3O$ 231.1372 [$M^+$], gefunden: 231.1368. **5-Ethyl-5,5a,6,7,8,9a,10-octahydro-pyrido[3,4-*b*] chinoxalin (29):** In 30 ml THF suspendiert man 0.619 g (4.64 mmol) $AlCl_3$ und läßt 45 min bei Raumtemperatur rühren. Anschließend kühlt man auf 0 °C und gibt 0.300 g (7.91 mmol) $LiAlH_4$ in kleinen Portionen hinzu. Nach weiteren rühren von 15 min wird Verbindung **28** (0.832 g, 3.59 mmol) zugegeben und 1 h bei 0 °C gerührt. Anschließend wird für 8 h auf Rückfluß erhitzt und nachdem abkühlen der Reaktionsmischung auf Eiswasser gegossen. Der anorganische Niederschlag wird abfiltriert und zweimal mit 30 ml DCM gewaschen. Die Mutterlauge würde auf pH 12 gebracht und dreimal mit 40 ml DCM extrahiert. Die kombiemden organischen Phasen werden über $Na_2SO_4$ getrocknet und das Lösungsmittel am Rotationsverdampfer abdestilliert. Das Rohprodukt wird durch Flashchromatographie an Kieselgel gereinigt ($EtOAc/NEt_3$, 10:1). Man erhält 0.430 g (1.98 mmol, 55%) eines weißen Schaums. $R_f$=0.24 ($EtOAc/NEt_3$, 10:1). $^1H$ NMR: (300 MHz, $CDCl_3$): δ = 1.06 (t, $^3J$= 7.2 Hz, 3H, $H_3$C-CH2-N-5), 1.25 (m, 2H), 1.54 (m, 5H), 1.78 (m, 1H), 3.20 (m, 4H), 3.79 (s, 1H, N-H), 6.21 (d, $^3J$= 5.7 Hz, 1H, H-4), 7.57 (s, H-1, H-1), 7.65 (d, $^3J$= 5.7 Hz, 1H, H-3). $^{13}C$ NMR (75 MHz, $CDCl_3$): δ = 12.1 ($H_3C$-$CH_2$-N-5), 19.3 (C-7), 24.8 (C-8), 26.8 (C-6), 30.9 (C-9), 42.8 ($H_3C$-$CH_2$-N-5), 47.8 (C-5a), 58.4 (C-9a), 103.9 (C-4), 129.8 ($C_q$, C-10a), 133.4 (C-1), 138.4 ($C_q$, C-10a), 140.9 (C-3) ppm. GC-MS(EI): RT 10.58 min, *m*/*z* (%) 218 (17), 217 ($M^+$, 100), 216 (7), 215 (7), 213 (5), 203 (5), 202 (51), 186 (17), 185 (5), 174 (12), 162 (6), 161 (8), 160 (9), 158 (7), 148 (5); 148 (5), 146 (16), 145 (7), 134 (8), 133 (9), 132 (25), 120 (14), 78 (4); IR (neat): □ = 3212 (m), 3093 (w), 2971 (w), 2928 (s), 2851 (s), 1589 (s), 1560 (m), 1505 (vs), 1470 (w), 1442 (m), 1419 (m), 1363 (s), 1280 (vs), 1244 (s), 1210 (m), 1194 (s), 1177 (m), 1108 (m), 1072 (m), 1039 (m), 1007 (w) 968 (w), 897 (w), 886 (w), 795 (s), 740 (m) $cm^{-1}$. HRMS (EI): berechnet für $C_{13}H_{19}N_3$ 217.1579 [$M^+$], gefunden: 215.1573.

**1-(5-Ethyl-5a,6,7,8,9,9a-hexahydro-5*H*-pyrido[3,4-*b*]chinoxalin-10-yl)-ethanon (30):** In einen 100 ml Schlenkkolben gibt man **29** (0.250 g, 1.15 mmol) und 10 ml THF. Die Lösung wird auf - 78 °C gekühhlt und 0.51 ml *n*-BuLi (1.27 mmol, 1.1 eq; 2.5 M in Hexan) zugegeben und die Reaktionslösung für 30 min ohne Kühlung gerührt. Nach 30 min wird die Reaktionslösung erneut auf -78 °C gekühlt und 0.09 ml (0.109 g, 1.4 mmol, p=1.1051 g/ml) Acetylchlorid zugegeben, das Kältebad wird entfernt und 1 h ohne Kühlung gerührt. Anschließend wird die Reaktion mit 4 ml gestoppt und dreimal mit 20 ml DCM extrahiert. Die kombinierten organischen Phasen werden über $Na_2SO_4$ getrocknet und das Lösungsmittel am Rotationsverdampfer abdestillliert. Das Rohprodukt wird an Kieselgel (EtOAc/MeOH, 10:2) gereinigt und man erhält 48 mg (0.18 mmol, 16 %) eines farblosen Feststoffs. $R_f$=0.16 ($EtOAc/NEt_3$, 10:1). $^1H$ NMR:(300 MHz, $CDCl_3$): δ = 1.19 (t, $^3J$= 7.2 Hz, 3H, $H_3$C-$CH_2$-N-5), 1.24 (m, 2H), 1.45 (m, 2H), 1.58 (m, 2H), 1.74 (m, 1H), 2.18 (m, 1H), 3.34 (m, $^2J$=14.4 Hz, $^3J$=7.2 Hz, 1H, $H_3$C-C$H_{AB}$-N-5), 3.53 (m, 1H, C-*H*), 3.57 (m, $^2J$=15.2 Hz, $^3J$=7.2 Hz, 1H, $H_3$C-C$H_{AB}$-N-5), 4.90 (bs, 1H, C-*H*), 6.62 (d, $^3J$=5.6 Hz, 1H, H-4), 8.12 (s, 1H, H-1), 8.08 (d, $^3J$=5.6 Hz, 1H, H-3). $^{13}C$ NMR (100 MHz, $CDCl_3$): δ = 12.4 ($H_3C$-$CH_2$-N-5), 18.9 (C-7), 22.8 ($H_3C$-CO-N-10), 24.7 (C-8), 25.8 (C-6), 28.5 (C-9), 40.0 ($H_3C$-$CH_2$-N-5), 48.7 (C-9a), 53.7 (C-5a), (C-4), 106.4 (C-4), 120.1 ($C_q$, C-10a), 145.1, 145.2 (C-1, $C_q$, C-4a), 147.1 (C-3), 167.1 (C=O) ppm. GC-MS(EI): RT 10.39 min, *m*/*z* (%) 260 (11), 259 ($M^+$, 100), 230 (8), 218 (9), 217 (79), 216 (40), 203 (4), 202 (23), 203 (3), 202 (23), 201 (4), 189 (6), 188 (31), 187 (4), 186 (3), 174 (11), 162 (4), 161 (6), 160 (5), 158 (4), 148 (5), 146 (6), 136 (3), 132 (4), 131 (12), 120 (6), 43 (3); IR (neat): □ = 3398 (s), 2933 (s), 2863 (m), 1731 (m), 1637 (vs), 1595 (vs), 1512 (s), 1546 (w), 1449 (m), 1397 (s), 1365 (s), 1334 (m), 1285 (s), 1239 (m), 1198 (m), 1168 (vs), 1123 (w), 1067 (w), 1068 (w), 1016 (w), 802 (vs), 718 (w), 668 (m). HRMS (EI): berechnet für $C_{15}H_{21}N_3O$ 259.1685 [$M^+$], gefunden: 259.1687.

**2,3-Diphenyl-pyrido[3,4-*b*]chinoxalin (25)** (modifizierte Vorschrift aus Lit.): Zu einer Suspension von 2.86 g (0.026 mol) 3,4-Diaminopyridin in 50 ml Ethanol werden 5.51 g (0.026 mmol) Benzil zugegeben und die Reaktionsmischung für 6 h auf 70 °C Ölbadtemperatur erhitzt. Nach Abkühlen auf Raumtemperatur wird der ausgefallene gelbe Feststoff abfiltriert (vielleicht ist das Kühlen mit einem Eiswasserbad nützlich). Umkristallisation aus Ethanol ergibt 6.91 g (0.024 mol, 94 %) eines schwach gelben Feststoffs. $^1H$ NMR:(400 MHz, $CDCl_3$): δ = 7.32-7.42 (m, 6H, 3,4,5-*phenyl*-H), 7.54-7.51(m, 4H, 2,6-*phenyl*-H), 7.98 (dd, $^3J$ = 6 Hz, $^4J$ = 0.8 Hz, 1H, 7-H), 8.82 (d, $^3J$ = 6 Hz, 1H, 8-H), 9.59 (d, $^4J$=0.8 Hz, 1H, 5-H) ppm. $^{13}C$ NMR (100 MHz, $CDCl_3$): δ = 121.3 (C-7), 128.4, 129.4-129.9 (*phenyl*-C), 136.3 ($C_q$, C-4a), 143.5 ($C_q$, C-8a), 147.3 (C-8), 154.5 (C-5), 155.3, 157.9 ($C_q$, *phenyl*-C) ppm. GC-MS(EI): RT 12.34 min, *m*/*z* (%) 285 (3), 284 ($M^+$+H, 22), 283 ($M^+$, 100), 282 (46), 206 (2), 181 (3), 180 (25), 179 (17), 154 (3), 153 (5), 152 (3) 142 (1), 141 (8), 140 (4), 127 (2), 104 (3), 103 (14), 102 (3), 78 (2), 77 (5), 76 (4), 51 (2), 50 (9); IR (neat): □ = 3061 (w), 1589 (m), 1577 (w), 1538 (w), 1492 (w), 1443 (m), 1419 (w), 1379 (s), 1346 (w), 1326 (m), 1315 (m), 1288 (w), 1211 (m), 1244 (w), 1227 (m), 1212 (w), 1180 (w), 1075 (m), 1058 (m), 1020 (m), 1001 (w), 976 (s), 921 (w), 892 (m), 892 (m), 830 (m), 819 (w), 811 (m), 763 (s), 735 (m), 708 (vs), 629 (s), 615 (m) $cm^{-1}$.

**2,3-Diphenyl-1,2,3,4-tetrahydro-pyrido[3,4-b]pyrazin (31):** In einen 500 ml Kolben gibt man **25** (6.25 g, 22.05 mmol) und 200 ml Ethanol. Zu der Lösung gibt man 6.25 g (165 mmol) gepulvertes $NaBH_4$ und rührt die Reaktionsmischung für 48 h bei 40 °C. Anschließend kühlt man auf Raumtemperatur ab und stoppt die Reaktion mit 10 ml kaltem Wasser. Nach weiteren rühren für 10 min wird die Reaktionsmischung zweimal mit 100 ml DCM extrahiert und die kombinierten organischen Phasen über $Na_2SO_4$ getrocknet. Das Rohprodukt wird durch Flashchromatographie an Kieselgel ($EtOAc/NEt_3$, 10:1) gereinigt. Man erhält einen schwach gelben Feststoff. $R_f$=0.23 ($EtOAc/NEt_3$, 10:1); $^1H$

NMR:(300 MHz, $CDCl_3$): δ = 3.99 (s, 1H, N-H), 4.59 (s, 2H, 2,3-H), 4.62 (s, 1H, N-H), 6.39 (d, $^3J$ = 5.4 Hz, 1H, 8-H), 6.80 (dd, $^4J$ = 1.2 Hz, $^3J$ = 9.3 Hz, 2H, 2,6-*phenyl*-H), 6.84 (dd, $^4J$ = 1.2 Hz, $^3J$ = 9.3 Hz, 2H, 2,6-*phenyl*-H), 7.12 (m, 6H, 3,4,5-*phenyl*-H) ppm. $^{13}C$ NMR (75 MHz, $CDCl_3$): δ = 58.6, 60.1 (C-2,3), 108.0 (C-8), 127,9-128.4 (*phenyl*-C), 129.8 (C-4a), 139.8 ($C_q$, *phenyl*-C), 140.0 ($C_q$, C-8a), 134.9 (C-5), 141.3 (C-7) ppm; MS(EI): *m*/*z* (%) 288 (22), 287 ($M^+$, 100), 286 (24), 285 (7), 284 (6), 283 (8), 282 (5), 211 (13), 210 (78), 209 (5), 208 (13), 197 (4), 196 (26), 181 (13), 179 (3), 127 (4), 104 (7), 92 (3), 91 (32), 77 (5); IR (neat): □ = 3215 (w), 2830 (w), 1596 (s), 1519 (s), 1493 (w), 1466 (w), 1452 (s), 1360 (m), 1290 (s), 1250 (m), 1231 (m), 1174 (s), 1120 (m), 1072 (m), 1050 (w), 1029 (w), 1005 (w), 989 (w), 950 (w), 905 (w), 844 (w), 810 (m), 770 (m), 723 (m), 675 (vs), 668 (w), 645 (w), 618 (m) $cm^{-1}$. HRMS (EI): berechnet für $C_{19}H_{17}N_3$ 287.1422 [$M^+$], gefunden: 287.1402.

**4-Methyl-2,3-diphenyl-1,2,3,4-tetrahydro-pyrido[3,4-b]pyrazin (32):** In einen 100 ml Rundkolben löst man unter Eiskühlung 0.600 g (2.08 mmol) von 31 in 15.5 ml (417.6 mmol, p=1.22 g/ml) Ameisensäure und gibt 5.1 ml Formaldehyd-Lösung (183.6 mmol, p=1.22 g/ml, <37 % in Wasser) hinzu. Anschließend entfernt man das Eisbad und erhitzt für 48 h auf 120 °C Ölbadtemperatur. Nach dem abkühlen auf 0 °C gibt unter Eiskühlung 50 %ige NaOH hinzu bis der pH-Wert der Mutterlauge auf 12 angestiegen ist. Anschließend extrahiert man dreimal mit 50 ml DCM und trocknet die vereinigten organischen Phasen über $Na_2SO_4$. Nachdem abdestillieren des Solvens am Rotationsverdampfer reinigt man das Rohprodukt an Kieselgel durch Flashchromatographie ($CHCl_3$/Isohexan/$NEt_3$, 10:2:1). Man erhält 0.564 g (1.87 mmol, 90 %) eines gelben Feststoffs. $R_f$=0.40 ($CHCl_3$/*iso*-hexane/$NEt_3$, 10:2:1). $^1H$ NMR:(400 MHz, $CDCl_3$): δ = 2.85 (s, 3H, C*H*$_3$), 4.42 (s, 1H, N-H), 4.44 (d, $^3J$ = 3.6 Hz, 1H, H-3), 4.95 (d, $^3J$ = 3.6 Hz, 1H, H-2), 6.50 (d, $^3J$ = 5.2 Hz, 1H, H-8), 6.64 (dd, $^4J$ = 2.8 Hz, $^3J$ = 9.2 Hz, 2H, 2,6-*phenyl*-H), 6.90 (m, 2H, 2,6-*phenyl*-H) 7.05 (m, 2H, *phenyl*-H), 7.16 (m, 4H, *phenyl*-H), 7.82 (s, 1H, H-5), 7.84 (d, $^3J$ = 5.2 Hz ppm. $^{13}C$ NMR (75 MHz, $CDCl_3$): δ = 37.2 (*C*H$_3$), 57.8 (C-3), 67.9 (C-2), 107.6 (C-8), 127.4-128.5 (*phenyl*-C), 131.3 (C-5), 131.6 ($C_q$, C-4a) 137.4, 138.8 ($C_q$, *phenyl*-C), 139.9 (C-7, C-8a) ppm. MS(EI): *m*/*z* (%) 302 (22), 301 ($M^+$, 100), 300 (5), 286 (5), 224 (10), 222 (4), 211 (13), 210 (87), 209 (3), 208 (6), 195 (11), 181 (6), 179 (3), 178 (3), 178 (3), 150 (7), 132 (3), 127 (3), 120 (3), 104 (4); 92 (3), 91 (31), 85 (4), 83 (6), 78 (4), 77 (5); IR (neat): □ = 3200 (w), 3158 (w), 3029 (w), 2948 (w), 2881 (w), 2824 (w), 1578 (s), 1516 (vs), 1491 (m), 1452 (m), 1421 (m), 1374 (w), 1353 (w), 1326 (w), 1294 (w), 1256 (m), 1235 (m), 1199 (w), 1157 (m), 1131 (m), 1103 (w), 1073 (m), 1054 (m), 1031 (m), 1013 (m), 969 (w), 922 (w), 842 (w), 810 (s), 766 (s), 755 (m), 733 (m), 641 (vs) $cm^{-1}$. HRMS (EI): berechnet für $C_{20}H_{19}N_3$ 301.1574 [$M^+$], gefunden: 301.1559.

**4-(Methyl-2,3-diphenyl-1,2,3,4-tetrahydro-pyrido[3,4-b]pyrazin-1-yl)-ethanon (33):** In einen 250 ml Schlenkkolben gibt man 2.90 g (9.62 mmol) von 32 und 100 ml MTBE. Die Lösung wird auf -78 °C gekühlt und 4.62 ml n-BuLi (11.50 mmol, 1.1 eq; 2.5 M in Hexan) werden in einer Zeit von 10 min zugegeben. Die Reaktionsmischung wird ohne Kühlung für 30 min gerührt. Anschließend kühlt man auf -78 °C ab und gibt 1.0 ml (0.96 g, 12.55 mmol) Acetylchlorid hinzu. Der Reaktionsmischung wird erlaubt auf Raumtemperatur zu kommen und nach weiteren rühren für 30 min wird die Reaktion durch Zugabe von 10 ml Wasser gestoppt. Die Mutterlauge wird wird dreimal mit DCM extrahiert und die vereinigten organischen Phasen über $Na_2SO_4$ getrocknet. Nach abdestillieren des Solvens am Rotationsverdampfer kann das Rohprodukt entweder durch Flashchromatographie ($CHCl_3$/Isohexan/$NEt_3$, 10:1:1) oder Umkristallisation mit Ethylacetat gereinigt werden. Man erhält 1.2 g (3.49 mmol, 36 %) eines weißen Feststoffs. $^1H$ NMR:(600 MHz, $CDCl_3$): δ = 2.40 (s, 3H, *H*$_3$C-CO-N), 2.86 (s, 3H, *H*$_3$C), 4.99 (m, 1H, H-3), 5.35 (d, $^3J$ = 4.2 Hz, 1H, H-2), 6.53 (d, $^3J$ = 7.8 Hz, 2H, 2,6-*phenyl*-H), 6.80 (bs, 2H, 2,6-*phenyl*-H), 7.08 (t, 2H, $^3J$ = 7.2 Hz, *phenyl*-H) 7.17 (t, $^3J$ = 6.0 Hz, 2H, *phenyl*-H), 7.22 (t, $^3J$ = 6.0 Hz, 1H, *phenyl*-H), 7.25 (t, $^3J$ = 6.0 Hz, 1H, *phenyl*-H), 8.03 (d, $^3J$ = 6.6 Hz, 1H, H-8), 8.17 (s, 1H, H-5), 8.41 (d, $^3J$ = 6.6 Hz, 1H, H-7) ppm. $^{13}C$ NMR (150 MHz, $CDCl_3$): δ = 24.60 ($H_3$C-CO-N), 36.9 ($H_3$*C*), 61.8 (C-3), 63.8 (C-2), 116.9 (C-8), 124.6 (C-5), 128.4-128.9 (*phenyl*-C), 134.7 ($C_q$, C-8a), 138.7 ($C_q$, C-4a),171.0 (*C*=O) ppm; GC-MS (EI): RT 1.22 min, *m*/*z* (%) 345 (3), 344 (27), 343 ($M^+$, 100), 315 (6), 302 (7), 301 (41), 300 (84), 224 (6), 223 (4), 222 (5), 211 (4), 210 (27), 208 (9), 195 (6), 181 (9), 179 (4), 178 (4), 146 (4), 118 (15), 104 (5); 92 (9), 91 (94), 78 (4), 77 (4), 43 (6); IR (neat): □ = 2428 (m), 2039 (w), 1688 (s), 1613 (w), 1534 (s), 1492 (m), 1454 (w), 1388 (m), 1364 (m), 1345 (m), 1317 (w), 1292 (w), 1292 (w), 1272 (w), 1227 (vs), 1209 (vs), 1122 (w), 1103 (w), 1078 (m), 1028 (m), 998 (m), 822 (s), 799 (m), 768 (m), 704 (m), 691 (m), 638 (w) $cm^{-1}$. HRMS (EI): berechnet für $C_{22}H_{23}N_3O$ 343.1685 [$M^+$], gefunden: 343.1699.

**1-Ethyl-4-methyl-2,3-diphenyl-1,2,3,4-tetrahydro-pyrido[3,4-b]pyrazin (34):** In 20 ml THF suspendiert man bei Raumtemperatur 0.575 g (4.320 mmol) $AlCl_3$ und rührt anschließend für 45 min bei dieser Temperatur. Anschließend kühlt man auf 0 °C ab und gibt 0.277 g (7,290 mmol) LiAlH4 in kleinen Portionen hinzu. Nach beendeter Zugabe wird die Reaktionsmischung für 15 min weiter gerührt und 33 (1.00 g, 3.32 mmol) zugegeben. Die Mischung wird für 1 h bei 0 °C gerührt und anschließend für 12 h auf Rückfluß erhitzt. Danach wird die Reaktionsmischung auf Raumtemperatur abgekühlt und auf Eiswasser gegossen. Der anorganische Niederschlag wird abfiltriert und die Mutterlauge mit DCM extrahiert. Die vereinigten organischen Phasen werden über $Na_2SO_4$ getrocknet und das erhaltene Rohprodukt durch Flashchromatographie (EtOAc/MeOH/$NEt_3$, 10/1/0.5) gereinigt. Man erhält 0.580 g (1.76 mmol, 53 %) eines gelben Feststoffs. $R_f$=0.57 (EtOAc/MeOH/NEt3, 10/1/0.5). $^1H$ NMR:(400 MHz, $CDCl_3$): δ = 1.05 (t, $^3J$ = 7.2 Hz, 3H, *H*$_3$C-CH$_2$-N), 2.71 (s, 3H, *H*$_3$C), 3.17 (qd, $^2J$ = 14.8 Hz, $^3J$ = 7.2 Hz, 1H, CH$_3$-C*H*$_{AB}$-N), 3.38 (qd, $^2J$ = 14.8 Hz, $^3J$ = 7.2 Hz, 1H, CH$_3$-C*H*$_{AB}$-N), 4.40 (d, $^3J$ = 3.6 Hz, 1H, H-3), 4.55 (d, $^3J$ = 3.6 Hz, 1H, H-2), 6.52 (d, 1H, $^3J$ = 5.6 Hz, H-8) 6.73 (m, 4H, *phenyl*-H), 7.07 (t, $^3J$ = 7.2 Hz, 2H, *phenyl*-H), 7.11 (t, $^3J$ = 8 Hz, 2H, *phenyl*-H), 7.16 (m, 2H, *phenyl*-H), 7.92 (s, 1H, H-5), 7.96 (d, $^3J$ = 5.6 Hz, 1H, H-7) ppm. $^{13}$C NMR (100 MHz, $CDCl_3$): δ = 11.1 ($H_3$*C*-$CH_2$-N), 37.1 ($H_3$*C*-N), 43.3 ($H_3C$-$CH_2$-N), 65.7 (C-2), 66.0 (C-3), 104.3 (C-8), 127.7-129.3 (*phenyl*-C), 133.0 (C-5), 133.3 ($C_q$, C-8a), 137.8, 138.6 ($C_q$, *phenyl*-C), 140.8 ($C_q$, C-8a), 141.0 (C-7) ppm. GC-HR-ESI-MS: RT 0.53-1.43 min, berechnet für $C_{44}H_{47}N_6$ 659.3862 [$2M^+$+H], gefunden: 659.3823, berechnet für $C_{22}H_{24}N_3$ 330.1970 [$M^+$+H] gefunden: 330.1940; IR (neat): □ = 2965 (w), 2818 (w), 1578 (s), 1518 (vs), 1492 (m), 1452 (s), 1430 (w), 1371 (m), 1356 (m), 1308 (m), 1286 (m), 1266 (s), 1241 (s), 1215 (vs), 1165 (m), 1119 (m), 1067 (s), 1023 (s), 884 (w), 867 (w), 828 (w), 810 (s), 764 (s), 749 (m), 705 (vs), 660 (w), 615 (w) $cm^{-1}$.

**Literaturangaben**

1 Für Übersichtsartikel siehe: (*a*) G. Höfle, W. Steglich, H. Vorbrüggen, Angew. Chem. 1978, 90, 602 - 615; Angew. Chem. Int. Ed. Engl. 1978, 17, 569 - 583; (*b*) E. F. V. Scriven, Chem. Soc. Rev. 1983, 12, 129 - 161; (*c*) A. Hassner, in Encyclopedia of Reagents for Organic Synthesis, Wiley, Chichester, 1995, 2022 - 2024; (*d*) U. Ragnarsson, L. Grehn, Acc. Chem. Res. 1998, 31, 494 - 501; (*e*) A. C. Spivey, A. Maddaford, A. Redgrave, Org. Prep. Proced. Int. 2000, 32, 331-365. (*f*) D. J. Berry, C. V. Digiovanna, S. S. Metrick, R. Murugan, Arkivoc 2001, 201 - 226; (*g*) A. C. Spivey, S. Arseniyadis, Angew. Chem. 2004, 116, 5552 - 5557; Angew. Chem. Int. Ed. Engl. 2004, 43, 5436 - 5441.

2 (*a*) E. Vedejs, M. Jure, Angew. Chem. 2005, 117, 4040 - 4069; Angew. Chem. Int. Ed. Engl. 2005, 44, 3971 - 4001. (*b*) P. I. Dalko, L. Moisan, Angew. Chem. 2004, 116, 5248 - 5286; Angew. Chem. Int. Ed. Engl. 2004, 43, 5138 - 5178. (*c*) G. Fu, Acc. Chem. Res. 2004, 37, 542 - 547. (*d*) S. France, D. J. Guerin, S. J. Miller, T. Lectka, Chem. Rev. 2003, 103, 2985 - 3012. (*e*) A. C. Spivey, A. Maddaford, A. Redgrave, Org. Prep. Proced. Int. 2000, 32, 331 - 365. (*f*) G. Fu, Acc. Chem. Res. 2000, 33, 412 - 420.

3 (*a*) T. Kawabata, R. Stragies, T. Fukaya, K. Fuji, Chirality 2003, 15, 71. (*b*) T. Kawabata, R. Stragies, T. Fukaya, Y. Nagaoka, H. Schedel, K. Fuji, Tetrahedron Lett. 2003, 44, 1545.

4 (*a*) G. Priem, B. Pelotier, S. J. F. Macdonald, M. S. Anson, I. B. Campbell, J. Org. Chem. 2003, 68, 3844. (*b*) B. Pelotier, G. Priem, S. J. F. Macdonald, M. S. Anson, R. J. Upton, I. B. Campbell, Tetrahedron Lett. 2005, 46, 9005.

5 (*a*) A. C. Spivey, T. Fekner, S. E. Spey, H. Adams, J. Org. Chem. 1999, 64, 9430, and literature cited there. (*b*) A. C. Spivey, T. Fekner, S. E. Spey, J. Org. Chem. 2000, 65, 3154. (*c*) A. C. Spivey, A. Maddafort, T. Fekner, A. J. Redgrave, C. S. Frampton, J. Chem. Soc. Perkin Trans. 1. 2000, 3460. (*d*) A. C. Spivey, A. Maddafort, T. Fekner, D. P. Leese, A. J. Redgrave, C. S. Frampton, J. Chem. Soc. Perkin Trans. 1, 2001, 1785. (*e*) C. Malardier-Jugroot, A. C. Spivey, M. A. Whitehead, J. Mol. Struct. (THEOCHEM) 2003, 623, 263. (*f*) A. C. Spivey, D. P. Leese, F. Zhu, S. G. Davey, R. L. Jarvest, Tetrahedron 2004, 60, 4513. (*g*) A. C. Spivey, S. Arseniyadis, T. Fekner, A. Maddaford, D. P. Lees, Tetrahedron 2006, 62, 295.

6 (*a*) C. O. Dalaigh, S. J. Hynes, D. J. Maher, S. J. Connon, Org. Biomol. Chem. 2005, 3, 981. (*b*) C. O. Dalaigh, S. J. Hynes, J. E. O'Brien, T. McCabe, D. J. Maher, G. W. Watson, S. J. Connon, Org. Biomol. Chem. 2006, 4, 2785.

7 (*a*) S. A. Shaw, P. Aleman, E. Vedejs, J. Am. Chem. Soc. 2003, 125, 13368-13369. (*b*) S. A. Shaw, P. Aleman, J. Christy, J. W. Kampf, P. Va, E. Vedejs, J. Am. Chem. Soc. 2006, 128, 925-934.

8 S. Yamada, T. Misono, Y. Iwai, Tet. Lett. 2005, 46, 2239.

9 (*a*) S. J. Miller, Acc. Chem. Res. 2004, 37, 601-610, and references therein. (*b*) M. B. Fierman, D. J. O'Leary, W. E. Steinmetz, S. J. Miller, J. Am. Chem. Soc. 2004, 126, 6967-6971. **(*c*)** J. W. Evans, M. B. Fierman, S. J. Miller, J. A. Ellman, J. Am. Chem. Soc. 2004, 126, 8134-8135. (*d*) B. R. Sculimbrene, Y. Xu, S. J. Miller, J. Am. Chem. Soc. 2004, 126, 13182-13183. (*e*) A. J. Morgan, Y. K. Wang, M. F. Roberts, S. J. Miller, J. Am. Chem. Soc. 2004, 126, 15370-15371. (*f*) C. A. Lewis, B. R. Sculimbrene, Y. Xu, S. J. Miller, Org. Lett. 2005, 3021. (*g*) Y. Xu, B. R. Sculimbrene, S. J. Miller, J. Org. Chem. 2006, 71, 4919-4928.

10 M. R. Heinrich, H. S. Klisa, H. Mayr, W. Steglich, H. Zipse, Angew. Chem. 2003, 115, 4975 - 4977; Angew. Chem. Int. Ed. 2003, 42, 4826 - 4828.

11. I.Held, A. Villinger, H. Zipse, Synthesis 2005, 1425 - 1426.

12 W. W. K. R. Mederski, D. Kux, M. Knoth, M. J. Schwarzkopf-Hofmann, Heterocycles, 2003, 4, 925-931.

13 J. Armand, L. Boulares, C. Bellec, J. Pinson, Can J. Chem. 1988, 66, 1500-1505.

14 M. L. Moore, Org. React. 1949, 5, 301-330.

15 C. Sotirou-Leventis, Z. Mao, A.-M. M. Rawashdeh, J. Org. Chem. 2000, 65, 6017-6023.

16 C. Kison, N. Meyer, T. Opatz, Angew. Chem. Int. Ed. 2005, 44, 5662-5664. Die dort zitierte Abhängigkeit der Reaktionsausbeute vom Alter der $BH_3$•THF-Lösung konnte für die Synthese von Verbindung **24** bestätigt werden.

17 Timothy D. W. Claridge, High-Resolution NMR Techniques in Organic Chemistry, Tetrahedron Organic Chemistry Series, 1999, 19, 298-299.

18 S. Xu, I. Held, B. Kempf, H. Mayr, W. Steglich, H. Zipse, Chem. Eur. J. 2005, 11, 4751 - 4757.

19 C. B. Fischer, S. Xu, H. Zipse, Chem. Eur. J. 2006, 12, 5779 - 5784.

20 Die Reaktionsgeschwindigkeit unterscheidet sich in diesem Fall kaum von der der Hintergrundreaktion. Dies macht eine genau Bestimmung der Halbwertszeiten mit den hier verwendeten Methoden ausgesprochen schwierig. Die in Tabelle 2 angegebenen Daten ergeben sich aus Umsatzmessungen für Reaktion A über 25 Tagen, 17 Stunden und 30 Min. und für Reaktion B über 31 Tage, 17 Stunden und 15 Min. In beiden Fällen ist ein Umsatz von 50% zu diesen Zeiten noch nicht erreicht.

21 Gaussian 03, Revision B.03, M. J. Frisch, G. W. Trucks, H. B. Schlegel, G. E. Scuseria, M. A. Robb, J. R. Cheeseman, J. A. Montgomery, Jr., T. Vreven, K. N. Kudin, J. C. Burant, J. M. Millam, S. S. Iyengar, J. Tomasi, V. Barone, B. Mennucci, M. Cossi, G. Scalmani, N. Rega, G. A. Petersson, H. Nakatsuji, M. Hada, M. Ehara, K. Toyota, R. Fukuda, J. Hasegawa, M. Ishida, T. Nakajima, Y. Honda, O. Kitao, H. Nakai, M. Klene, X. Li, J. E. Knox, H. P. Hratchian, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, P. Y. Ayala, K. Morokuma, G. A. Voth, P. Salvador, J. J. Dannenberg, V. G. Zakrzewski, S. Dapprich, A. D. Daniels, M. C. Strain, O.

Farkas, D. K. Malick, A. D. Rabuck, K. Raghavachari, J. B. Foresman, J. V. Ortiz, Q. Cui, A. G. Baboul, S. Clifford, J. Cioslowski, B. B. Stefanov, G. Liu, A. Liashenko, P. Piskorz, I. Komaromi, R. L. Martin, D. J. Fox, T. Keith, M. A. Al-Laham, C. Y. Peng, A. Nanayakkara, M. Challacombe, P. M. W. Gill, B. Johnson, W. Chen, M. W. Wong, C. Gonzalez, and J. A. Pople, Gaussian, Inc., Wallingford CT, **2004.**

22 H. E. Gottlieb, V. Kotyar, A. Nudelman, J. Org. Chem. 1997, 21 7512-7515.

23 Der Umsatz dieser Reaktion stoppt bei 59%. ESI-MS-Studien der Reaktionsmischung weisen darauf hin, daß der Katalysator zu dieser Zeit nicht mehr intakt ist.

**Patentansprüche**

1. 3,4-Diaminopyridine derivative of the formula I below

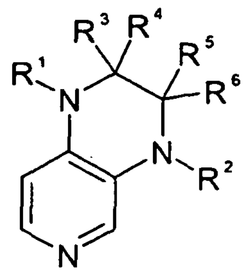

I

where
$R^1$ and $R^2$ are each, independently of one another, electron donors, with $R^2$ also being able to be H; and
$R^3$, $R^4$, $R^5$ and $R^6$ are each selected independently from among H, substituted or unsubstituted, straight-chain or branched alkyl, alkenyl, alkynyl, alkoxy and substituted or unsubstituted aryl, with the radicals $R^3$ and/or $R^4$ together with the radicals $R^5$ and/or $R^6$ being able to form a ring.

2. 3,4-Diaminopyridine derivative according to Claim 1, wherein $R^1$ and $R^2$ are each selected independently from among substituted or unsubstituted, straight-chain or branched alkyl, alkenyl, alkynyl, acyl, alkoxy and substituted or unsubstituted aryl, more preferably substituted or unsubstituted, straight-chain or branched $C_1$-$C_{20}$-alkyl, $C_2$-$C_{20}$-alkenyl, $C_2$-$C_{20}$-alkynyl, $C_1$-$C_{20}$-acyl, $C_1$-$C_{20}$-alkoxy, substituted or unsubstituted phenyl and benzyl, with $R^2$ also being able to be H.

3. 3,4-Diaminopyridine derivative according to Claim 1 or 2, wherein $R^3$, $R^4$, $R^5$ and $R^6$ are each selected independently from among H, substituted or unsubstituted, straight-chain or branched $C_1$-$C_{20}$-alkyl, $C_2$-$C_{20}$-alkenyl, $C_2$-$C_{20}$-alkynyl and substituted or unsubstituted phenyl and benzyl.

4. 3,4-Diaminopyridine derivative according to any of the preceding claims, wherein at least one of the radicals $R^3$,

$R^4$, $R^5$ and $R^6$ is not H, with preference being given to at least one of the radicals $R^3$, $R^4$, $R^5$ and $R^6$ being phenyl or the two radicals $R^3$ and $R^5$ each being H and the two radicals $R^4$ and $R^6$ together being 1,4-butanediyl so as to form a 6-membered ring.

**5.** 3,4-Diaminopyridine derivative according to any of the preceding claims, wherein the two radicals $R^4$ and $R^5$ are located in a cis position relative to one another so as to give a 3,4-diaminopyridine derivative of the formula Ia:

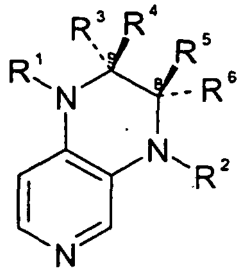

Ia

where
$R^1$ and $R^2$ are each, independently of one another, electron donors, with $R^2$ also being able to be H;
$R^3$ and $R^6$ are each selected independently from among H, substituted or unsubstituted, straight-chain or branched alkyl, alkenyl, alkynyl, alkoxy and substituted or unsubstituted aryl;
$R^4$ and $R^5$ are each selected independently from among substituted or unsubstituted, straight-chain or branched alkyl, alkenyl, alkynyl, alkoxy and substituted or unsubstituted aryl; and
the radicals $R^3$ and/or $R^4$ together with the radicals $R^5$ and/or $R^6$ can form a ring.

**6.** 3,4-Diaminopyridine derivative according to any of the preceding claims, wherein $R^1$ and $R^2$ are each, independently of one another, a substituted or unsubstituted, straight-chain or branched $C_1$-$C_{10}$-alkyl, preferably a substituted or unsubstituted, straight-chain or branched $C_2$-$C_8$-alkyl and most preferably a substituted or unsubstituted, straight-chain or branched $C_3$-$C_6$-alkyl.

**7.** 3,4-Diaminopyridine derivative according to any of the preceding claims, wherein $R^1$ and $R^2$ are each ethyl, $R^3$ and $R^6$ are each H and $R^4$ and $R^5$ together are 1,4-butanediyl so as to form a 6-membered ring.

**8.** Process for preparing a 3,4-diaminopyridine derivative of the formula I, which comprises the steps:

- reaction of 3,4-diaminopyridine with a 1,2-dicarbonyl compound;
- reduction of the resulting diimine to the corresponding diamine; and
- replacement of at least the hydrogen atom on the nitrogen in the 4 position of the pyridine ring.

**9.** Process according to claim 8, wherein both the nitrogen atoms in positions 3 and 4 of the pyridine ring are substituted.

**10.** Process according to Claim 8 or 9, wherein alkyl substitution is effected by means of a two-stage synthesis comprising acylation of the nitrogen atom and subsequent reduction of the resulting amide to an amine.

**11.** Use of a 3,4-diaminopyridine derivative according to any of Claims 1-7 as catalyst in a chemical reaction.

**12.** Use according to Claim 11, wherein the chemical reaction is an acylation reaction, in particular an acylation reaction of an alcohol or an amine, or a urethane and/or polyurethane synthesis.

**13.** Process for preparing an acylated alcohol or amine, wherein a 3,4-diaminopyridine derivative according to any of Claims 1-7 is added as catalyst in addtion to the reagents necessary for an acylation.

**14.** Process for preparing polyurethane, wherein a 3,4-diaminopyridine derivative according to any of Claims 1-7 is added as catalyst in addition to the reagents necessary for the preparation of the urethane and/or polyurethane.

**15.** Pharmaceutical composition or herbicide composition comprising a 3,4-diaminopyridine derivative according to any of Claims 1-7.

## Claims

**1.** 3,4-Diaminopyridin-Derivat der nachstehenden Formel I:

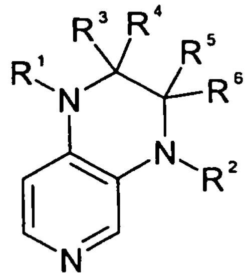

I

worin
$R^1$ und $R^2$ jeweils unabhängig voneinander Elektronendonatoren sind, wobei $R^2$ auch H sein kann; und
$R^3$, $R^4$, $R^5$ und $R^6$ jeweils unabhängig voneinander aus H, substituiertem oder unsubstituiertem, unverzweigtem oder verzweigtem Alkyl, Alkenyl, Alkinyl und Alkoxy sowie substituiertem oder unsubstituiertem Aryl ausgewählt sind, wobei die Reste $R^3$ und/oder $R^4$ zusammen mit den Resten $R^5$ und/oder $R^6$ einen Ring bilden können.

**2.** 3,4-Diaminopyridin-Derivat nach Anspruch 1, worin $R^1$ und $R^2$ jeweils unabhängig voneinander aus substituiertem oder unsubstituiertem, unverzweigtem oder verzweigtem Alkyl, Alkenyl, Alkinyl, Acyl und Alkoxy sowie substituiertem oder unsubstituiertem Aryl, noch bevorzugter substituiertem oder unsubstituiertem, unverzweigtem oder verzweigtem $C_1$-$C_{20}$-Alkyl, $C_2$-$C_{20}$-Alkenyl, $C_2$-$C_{20}$-Alkinyl, $C_1$-$C_{20}$-Acyl, $C_1$-$C_{20}$-Alkoxy, substituiertem oder unsubstituiertem Phenyl und Benzyl, ausgewählt sind, wobei $R^2$ auch H sein kann.

**3.** 3,4-Diaminopyridin-Derivat nach Anspruch 1 oder 2, worin $R^3$, $R^4$, $R^5$ und $R^6$ jeweils unabhängig voneinander aus H, substituiertem oder unsubstituiertem, unverzweigtem oder verzweigtem $C_1$-$C_{20}$-Alkyl, $C_2$-$C_{20}$-Alkenyl und $C_2$-$C_{20}$-Alkinyl sowie substituiertem oder unsubstituiertem Phenyl und Benzyl ausgewählt sind.

**4.** 3,4-Diaminopyridin-Derivat nach einem der vorangegangenen Ansprüche, worin zumindest einer der Reste $R^3$, $R^4$, $R^5$ und $R^6$ nicht H ist, wobei vorzugsweise zumindest einer der Reste $R^3$, $R^4$, $R^5$ und $R^6$ Phenyl ist, oder die beiden Reste $R^3$ und $R^5$ jeweils H sind und die beiden Reste $R^4$ und $R^6$ zusammen 1,4-Butandiyl sind, was einen 6-gliedrigen Ring ergibt.

**5.** 3,4-Diaminopyridin-Derivat nach einem der vorangegangenen Ansprüche, worin die beiden Reste $R^4$ und $R^5$ in cis-Position zueinander stehen, was das 3,4-Diaminopyridin-Derivat der Formel Ia ergibt:

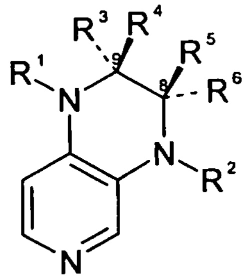

Ia

worin
$R^1$ und $R^2$ jeweils unabhängig voneinander Elektronendonatoren sind, wobei $R^2$ auch H sein kann; und
$R^3$ und $R^6$ jeweils unabhängig voneinander aus H, substituiertem oder unsubstituiertem, unverzweigtem oder verzweigtem Alkyl, Alkenyl, Alkinyl und Alkoxy sowie substituiertem oder unsubstituiertem Aryl ausgewählt sind;
$R^4$ und $R^5$ jeweils unabhängig voneinander aus substituiertem oder unsubstituiertem, unverzweigtem oder verzweigtem Alkyl, Alkenyl, Alkinyl und Alkoxy sowie substituiertem oder unsubstituiertem Aryl ausgewählt sind; und
die Reste $R^3$ und/oder $R^4$ zusammen mit den Resten $R^5$ und/oder $R^6$ einen Ring bilden können.

**6.** 3,4-Diaminopyridin-Derivat nach einem der vorangegangenen Ansprüche, worin $R^1$ und $R^2$ jeweils unabhängig voneinander substituiertes oder unsubstituiertes, unverzweigtes oder verzweigtes $C_1$-$C_{10}$-Alkyl, vorzugsweise substituiertes oder unsubstituiertes, unverzweigtes oder verzweigtes $C_2$-$C_8$-Alkyl, insbesondere substituiertes oder unsubstituiertes, unverzweigtes oder verzweigtes $C_3$-$C_6$-Alkyl, sind.

**7.** 3,4-Diaminopyridin-Derivat nach einem der vorangegangenen Ansprüche, worin $R^1$ und $R^2$ jeweils Ethyl sind, $R^3$ und $R^6$ jeweils H sind und $R^4$ und $R^5$ zusammen 1,4-Butandiyl sind, was einen 6-gliedrigen Ring ergibt.

**8.** Verfahren zur Herstellung eines 3,4-Diaminopyridin-Derivats der Formel I, das folgende Schritte umfasst:

- Umsetzen von 3,4-Diaminopyridin mit einer 1,2-Dicarbonylverbindung;
- Reduzieren des resultierenden Diimins zum entsprechenden Diamin; und
- Ersetzen zumindest des Wasserstoffatoms am Stickstoff an Position 4 des Pyridins.

**9.** Verfahren nach Anspruch 8, worin die beiden Stickstoffatome an Position 3 und 4 des Pyridinrings substituiert werden.

**10.** Verfahren nach Anspruch 8 oder 9, worin eine Alkylsubstitution mittels einer zweistufigen Synthese durchgeführt wird, welche die Acylierung des Stickstoffatoms und die nachfolgende Reduktion des resultierenden Amids zu einem Amin umfasst.

**11.** Verwendung eines 3,4-Diaminopyridin-Derivats nach einem der Ansprüche 1 bis 7 als Katalysator in einer chemischen Reaktion.

**12.** Verwendung nach Anspruch 11, worin die chemische Reaktion eine Acylierungsreaktion, insbesondere eine Acylierungsreaktion eines Alkohols oder eines Amins, oder die Synthese eines Urethans und/oder Polyurethans ist.

**13.** Verfahren zur Herstellung eines acylierten Alkohols oder Amins, worin zusätzlich zu dem für eine Acylierung erforderlichen Reagens ein 3,4-Diaminopyridin-Derivat nach einem der Ansprüche 1 bis 7 als Katalysator zugesetzt wird.

**14.** Verfahren zur Herstellung von Polyurethan, worin zusätzlich zu dem fur die Herstellung des Urethans und/oder Polyurethans erforderlichen Reagens ein 3,4-Diaminopyridin-Derivat nach einem der Ansprüche 1 bis 7 als Katalysator zugesetzt wird.

**15.** Pharmazeutische Zusammensetzung oder Herbizidzusammensetzung, die ein 3,4-Diaminopyridin-Derivat nach einem der Ansprüche 1 bis 7 umfasst.

**Revendications**

**1.** Dérivé de 3,4-diaminopyridine de la formule I ci-dessous :

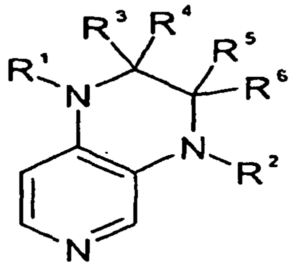

I

dans laquelle
$R^1$ et $R^2$ sont chacun indépendamment l'un de l'autre des donneurs d'électrons, avec $R^2$ pouvant également être H ; et $R^3$, $R^4$, $R^5$ et $R^6$ sont chacun choisis indépendamment parmi H, un groupe alkyle, alcényle, alcynyle, alcoxy linéaire ou ramifié, substitué ou non substitué ou aryle substitué ou non substitué, avec les radicaux $R^3$ et/ou $R^4$ pouvant former en association avec les radicaux $R^5$ et/ou $R^6$ un noyau.

**2.** Dérivé de 3,4-diaminopyridine selon la revendication 1, dans lequel $R^1$ et $R^2$ sont chacun choisis indépendamment parmi un groupe alkyle, alcényle, alcynyle, acyle, alcoxy linéaire ou ramifié, substitué ou non substitué et aryle substitué ou non substitué, encore mieux un groupe alkyle en $C_1$-$C_{20}$, alcényle en $C_2$-$C_{20}$, alcynyle en $C_2$-$C_{20}$, acyle en $C_1$-$C_{20}$, alcoxy en $C_1$-$C_{20}$ linéaire ou ramifié, substitué ou non substitué, phényle ou benzyle substitué ou non substitué avec $R^2$ pouvant également être H.

**3.** Dérivé de 3,4-diaminopyridine selon la revendication 1 ou 2, dans lequel $R^3$, $R^4$, $R^5$ et $R^6$ sont chacun choisis indépendamment parmi H, un groupe alkyle en $C_1$-$C_{20}$, alcényle en $C_2$-$C_{20}$, alcynyle en $C_2$-$C_{20}$ linéaire ou ramifié, substitué ou non substitué et phényle et benzyle substitués ou non substitués.

**4.** Dérivé de 3,4-diaminopyridine selon l'une quelconque des revendications précédentes, dans lequel au moins un des radicaux $R^3$ , $R^4$, $R^5$ et $R^6$ n'est pas H, avec une préférence donnée à au moins un des radicaux $R^3$, $R^4$, $R^5$ et $R^6$ étant le groupe phényle ou les deux radicaux $R^3$ et $R^5$ étant chacun H et les deux radicaux $R^4$ et $R^5$ étant ensemble le groupe 1,4-butanediyle afin de former un noyau à 6 éléments.

**5.** Dérivé de 3,4-diaminopyridine selon l'une quelconque des revendications précédentes, dans lequel les deux radicaux $R^4$ et $R^5$ sont disposés dans une position cis l'un par rapport à l'autre afin de fournir un dérivé de 3,4-diaminopyridine de la formule Ia :

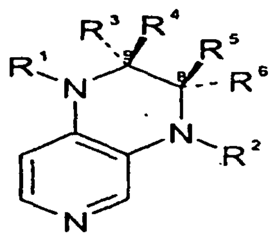

Ia

dans laquelle
$R^1$ et $R^2$ sont chacun indépendamment l'un de l'autre des donneurs d'électrons, avec $R^2$ pouvant également être H ;
$R^3$ et $R^6$ sont chacun choisis indépendamment parmi H, un groupe alkyle, alcényle, alcynyle, alcoxy linéaire ou ramifié, substitué ou non substitué et aryle substitué ou non substitué ;
$R^4$ et $R^5$ sont chacun choisis indépendamment parmi un groupe alkyle, alcényle, alcynyle, alcoxy linéaire ou ramifié, substitué ou non substitué et aryle substitué ou non substitué ; et
les radicaux $R^3$ et/ou $R^4$ avec les radicaux $R^5$ et/ou $R^6$ peuvent former un noyau.

**6.** Dérivé de 3,4-diaminopyridine selon l'une quelconque des revendications précédentes, dans lequel $R^1$ et $R^2$ sont chacun, indépendamment l'un de l'autre, un groupe alkyle en $C_1$-$C_{10}$ linéaire ou ramifié, substitué ou non substitué, de préférence un groupe alkyle en $C_2$-$C_8$ linéaire ou ramifié, substitué ou non substitué et bien mieux encore un groupe alkyle en $C_3$-$C_6$ linéaire ou ramifié, substitué ou non substitué.

**7.** Dérivé de 3,4-diaminopyridine selon l'une quelconque des revendications précédentes, dans lequel $R^1$ et $R^2$ sont chacun le groupe éthyle, $R^3$ et $R^6$ sont chacun H et $R^4$ et $R^5$ forment ensemble le groupe 1,4-butanediyle afin de former un noyau à 6 éléments.

**8.** Procédé de préparation d'un dérivé de 3,4-diaminopyridine de la formule I, lequel comprend les étapes :

- de réaction de 3,4-diaminopyridine avec un composé 1,2-dicarbonyle ;
- de réduction de la diimine résultante en la diamine correspondante ; et
- de remplacement d'au moins l'atome d'hydrogène sur l'azote dans la position 4 du noyau pyridine.

**9.** Procédé selon la revendication 8, dans lequel les deux atomes d'azote aux positions 3 et 4 du noyau pyridine sont substitués.

**10.** Procédé selon la revendication 8 ou 9, dans lequel une substitution alkyle est réalisée au moyen d'une synthèse en deux étapes comprenant l'acylation de l'atome d'azote et la réduction subséquente de l'amide résultant en une amine.

**11.** Utilisation d'un dérivé de 3,4-diaminopyridine selon l'une quelconque des revendications 1-7 comme catalyseur dans une réaction chimique.

**12.** Utilisation selon la revendication 11, dans laquelle la réaction chimique est une réaction d'acylation, en particulier une réaction d'acylation d'un alcool ou d'une amine, ou une synthèse d'uréthane et/ou de polyuréthane.

**13.** Procédé de préparation d'un alcool ou d'une amine acylé, dans lequel un dérivé de 3,4-diaminopyridine selon l'une quelconque des revendications 1-7 est ajouté comme catalyseur en plus des réactifs nécessaires pour une acylation.

**14.** Procédé de préparation de polyuréthane, dans lequel un dérivé de 3,4-diaminopyridine selon l'une quelconque des revendications 1-7 est ajouté comme catalyseur en plus des réactifs nécessaires pour la préparation de l'uréthane et/ou du polyuréthane.

**15.** Composition pharmaceutique ou composition herbicide comprenant un dérivé de 3,4-diaminopyridine selon l'une quelconque des revendications 1-7.

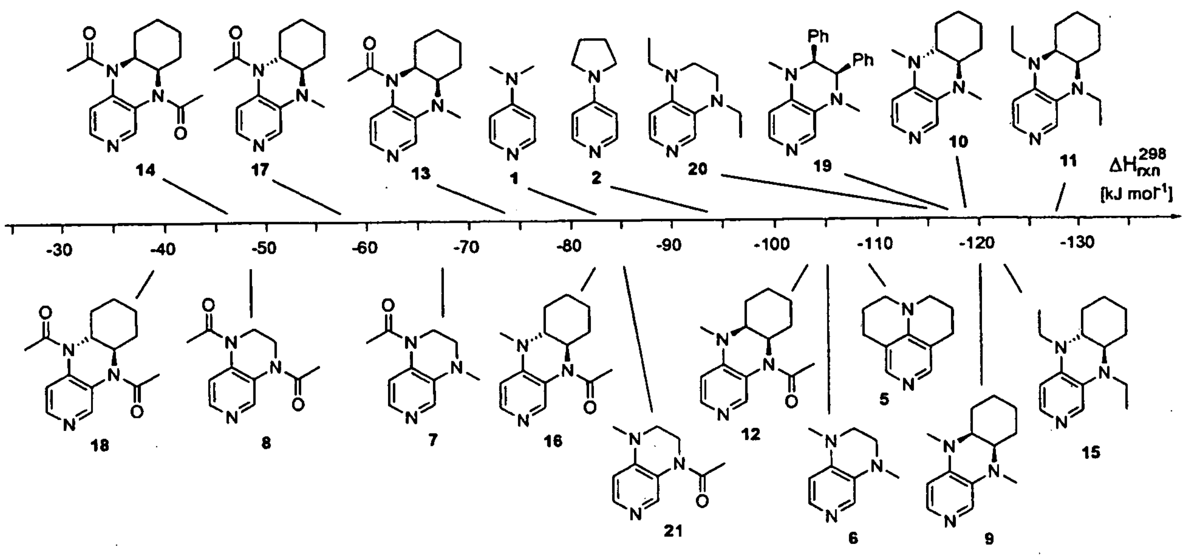
14
17
13
1
2
20
19
10
11
$\Delta H_{rxn}^{298}$
[kJ mol$^{-1}$]
-30
-40
-50
-60
-70
-80
-90
-100
-110
-120
-130
18
8
7
16
21
12
5
6
9
15

Fig. 1

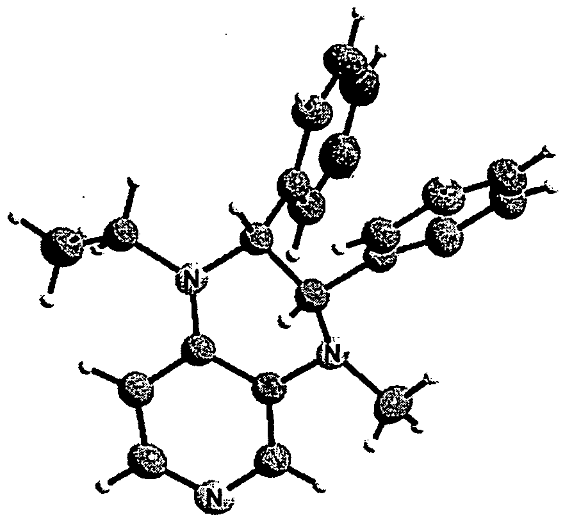

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **G. Höfle ; W. Steglich ; H. Vorbrüggen.** *Angew. Chem.,* 1978, vol. 90, 602-615
- *Angew. Chem. Int. Ed. Engl.,* 1978, vol. 17, 569-583
- **E. F. V. Scriven.** *Chem. Soc. Rev.,* 1983, vol. 12, 129-161
- **A. Hassner.** Encyclopedia of Reagents for Organic Synthesis. Wiley, 1995, 2022-2024
- **U. Ragnarsson ; L. Grehn.** *Acc. Chem. Res.,* 1998, vol. 31, 494-501
- **A. C. Spivey ; A. Maddaford ; A. Redgrave.** *Org. Prep. Proced. Int.,* 2000, vol. 32, 331-365
- **D. J. Berry ; C. V. Digiovanna ; S. S. Metrick ; R. Murugan.** *Arkivoc,* 2001, 201-226
- **A. C. Spivey ; S. Arseniyadis.** *Angew. Chem.,* 2004, vol. 116, 5552-5557
- *Angew. Chem. Int. Ed. Engl.,* 2004, vol. 43, 5436-5441
- **E. Vedejs ; M. Jure.** *Angew. Chem.,* 2005, vol. 117, 4040-4069
- *Angew. Chem. Int. Ed. Engl.,* 2005, vol. 44, 3971-4001
- **P. I. Dalko ; L. Moisan.** *Angew. Chem.,* 2004, vol. 116, 5248-5286
- *Angew. Chem. Int. Ed. Engl.,* 2004, vol. 43, 5138-5178
- **G. Fu.** *Acc. Chem. Res.,* 2004, vol. 37, 542-547
- **S. France ; D. J. Guerin ; S. J. Miller ; T. Lectka.** *Chem. Rev.,* 2003, vol. 103, 2985-3012
- **A. C. Spivey ; A. Maddaford ; A. Redgrave.** *Org. Prep. Proced. Int,* 2000, vol. 32, 331-365
- **G. Fu.** *Acc. Chem. Res.,* 2000, vol. 33, 412-420
- **T. Kawabata ; R. Stragies ; T. Fukaya ; K. Fuji.** *Chirality,* 2003, vol. 15, 71
- **T. Kawabata ; R. Stragies ; T. Fukaya ; Y. Nagaoka ; H. Schedel ; K. Fuji.** *Tetrahedron Lett.,* 2003, vol. 44, 1545
- **G. Priem ; B. Pelotier ; S. J. F. Macdonald ; M. S. Anson ; I. B. Campbell.** *J. Org. Chem.,* 2003, vol. 68, 3844
- **B. Pelotier ; G. Priem ; S. J. F. Macdonald ; M. S. Anson ; R. J. Upton ; I. B. Campbell.** *Tetrahedron Lett.,* 2005, vol. 46, 9005
- **A. C. Spivey ; T. Fekner ; S. E. Spey ; H. Adams.** *J. Org. Chem.,* 1999, vol. 64, 9430
- **A. C. Spivey ; T. Fekner ; S. E. Spey.** *J. Org. Chem.,* 2000, vol. 65, 3154
- **A. C. Spivey ; A. Maddafort ; T. Fekner ; A. J. Redgrave ; C. S. Frampton.** *J. Chem. Soc. Perkin Trans.,* 2000, vol. 1, 3460
- **A. C. Spivey ; A. Maddafort ; T. Fekner ; D. P. Leese ; A. J. Redgrave ; C. S. Frampton.** *J. Chem. Soc. Perkin Trans.,* 2001, vol. 1, 1785
- **C. Malardier-Jugroot ; A. C. Spivey ; M. A. Whitehead.** *J. Mol. Struct. (THEOCHEM),* 2003, vol. 623, 263
- **A. C. Spivey ; D. P. Leese ; F. Zhu ; S. G. Davey ; R. L. Jarvest.** *Tetrahedron,* 2004, vol. 60, 4513
- **A. C. Spivey ; S. Arseniyadis ; T. Fekner ; A. Maddaford ; D. P. Lees.** *Tetrahedron,* 2006, vol. 62, 295
- **C. O. Dalaigh ; S. J. Hynes ; D. J. Maher ; S. J. Connon.** *Org. Biomol. Chem.,* 2005, vol. 3, 981
- **C. O. Dalaigh ; S. J. Hynes ; J. E. O'Brien ; T. McCabe ; D. J. Maher ; G. W. Watson ; S. J. Connon.** *Org. Biomol. Chem.,* 2006, vol. 4, 2785
- **S. A. Shaw ; P. Aleman ; E. Vedejs.** *J. Am. Chem. Soc.,* 2003, vol. 125, 13368-13369
- **S. A. Shaw ; P. Aleman ; J. Christy ; J. W. Kampf ; P. Va ; E. Vedejs.** *J. Am. Chem. Soc.,* 2006, vol. 128, 925-934
- **Yamada, T. Misono ; Y. Iwai.** *Tet. Lett.,* 2005, vol. 46, 2239
- **S. J. Miller.** *Acc. Chem. Res.,* 2004, vol. 37, 601-610
- **M. B. Fierman ; D. J. O'Leary ; W. E. Steinmetz ; S. J. Miller.** *J. Am. Chem. Soc.,* 2004, vol. 126, 6967-6971
- **J. W. Evans ; M. B. Fierman ; S. J. Miller ; J. A. Ellman.** *J. Am. Chem. Soc.,* 2004, vol. 126, 8134-8135
- **B. R. Sculimbrene ; Y. Xu ; S. J. Miller.** *J. Am. Chem. Soc.,* 2004, vol. 126, 13182-13183
- **A. J. Morgan ; Y. K. Wang ; M. F. Roberts ; S. J. Miller.** *J. Am. Chem. Soc.,* 2004, vol. 126, 15370-15371
- **C. A. Lewis ; B. R. Sculimbrene ; Y. Xu ; S. J. Miller.** *Org. Lett.,* 2005, 3021
- **Y. Xu ; B. R. Sculimbrene ; S. J. Miller.** *J. Org. Chem.,* 2006, vol. 71, 4919-4928
- **M. R. Heinrich ; H. S. Klisa ; H. Mayr ; W. Steglich ; H. Zipse.** *Angew. Chem.,* 2003, vol. 115, 4975-4977

- *Angew. Chem. Int. Ed.,* 2003, vol. 42, 4826-4828
- **I.Held, A. Villinger ; H. Zipse.** *Synthesis,* 2005, 1425-1426
- **W. W. K. R. Mederski ; D. Kux ; M. Knoth ; M. J. Schwarzkopf-Hofmann.** *Heterocycles,* 2003, vol. 4, 925-931
- **J. Armand ; L. Boulares ; C. Bellec ; J. Pinson.** *Can J. Chem.,* 1988, vol. 66, 1500-1505
- **M. L. Moore.** *Org. React.,* 1949, vol. 5, 301-330
- **C. Sotirou-Leventis ; Z. Mao ; A.-M. M. Rawashdeh.** *J. Org. Chem.,* 2000, vol. 65, 6017-6023
- **C. Kison ; N. Meyer ; T. Opatz.** *Angew. Chem. Int. Ed.,* 2005, vol. 44, 5662-5664
- **Timothy D. W. Claridge.** *High-Resolution NMR Techniques in Organic Chemistry, Tetrahedron Organic Chemistry Series,* 1999, vol. 19, 298-299
- **S. Xu ; I. Held ; B. Kempf ; H. Mayr ; W. Steglich ; H. Zipse.** *Chem. Eur. J.,* 2005, vol. 11, 4751-4757
- **C. B. Fischer ; S. Xu ; H. Zipse.** *Chem. Eur. J.,* 2006, vol. 12, 5779-5784
- **H. E. Gottlieb ; V. Kotyar ; A. Nudelman.** *J. Org. Chem.,* 1997, vol. 21, 7512-7515